(19) Europäisches Patentamt
European Patent Office
Office européen des brevets

(11) Publication number: **0 352 863 A2**

(12) **EUROPEAN PATENT APPLICATION**

(21) Application number: **89201956.3**

(51) Int. Cl.4: **C07D 211/90 , A61K 31/445**

(22) Date of filing: **25.07.89**

Claims for the following Contracting States: ES + GR.

The title of the invention has been amended (Guidelines for Examination in the EPO, A-III, 7.3).

(30) Priority: **29.07.88 IT 2154188**
**29.07.88 IT 2154588**

(43) Date of publication of application:
**31.01.90 Bulletin 90/05**

(84) Designated Contracting States:
**AT BE CH DE ES FR GB GR IT LI LU NL SE**

(71) Applicant: **SIMES, SOCIETA ITALIANA MEDICINALI E SINTETICI, S.P.A.**
**Via della Chimica 9**
**I-36100 Vicenza(IT)**

(72) Inventor: **Casagrande, Cesare**
**Via Campogalio, 21/67**
**I-20020 Arese (Milano)(IT)**
Inventor: **Montanari, Stefania**
**Via Martinengo, 1**
**I-20139 Milano(IT)**
Inventor: **Santangelo, Francesco**
**Via Don Gnocchi, 33**
**I-20148 Milano(IT)**

(74) Representative: **Marchi, Massimo et al**
**c/o Marchi & Mittler s.r.l. Viale Lombardia 20**
**I-20131 Milano(IT)**

(54) **1,4-Dihydropyridine dicarboxylic acid derivatives.**

(57) Compounds of formula

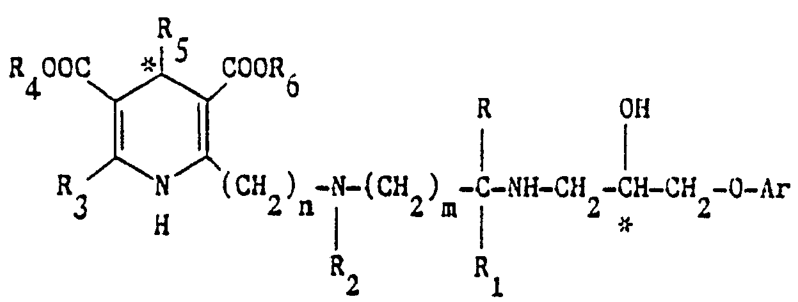

(I)

(wherein Ar, R, $R_1$, $R_2$, $R_3$, $R_4$, $R_5$, m and n have the meanings given in the description), their preparation and pharmaceutical compositions containing them.

The compounds of formula I are active on the cardiovascular system as antivasospastics, antiangina agents, antihypertensives and vasodilators.

EP 0 352 863 A2

## Compounds active on the cardiovascular system

The present invention relates to compounds active on the cardiovascular system; more particularly, it relates to compounds having a dihydropyridine nucleus, pharmaceutically acceptable salts thereof, processes for preparing them and pharmaceutical compositions containing them.

Compounds having calcium-antagonist properties and 4-aryldihydropyridine structure are known; their typical representative is 4-(2-nitrophenyl)-2,6-dimethyl-3,5-dimethoxycarbonyl)-1,4-dihydropyridine known as Nifepidine (Merck Index 10th Ed., No. 6374) of formula

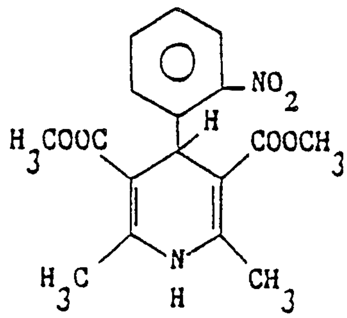

(A)

Beta-blocking drugs are also known and widely used in therapy for treating cardiovascular diseases. in most cases these drugs are structurally related to 3-aryloxy-2-hydroxy-propanamines.

Examples of beta-blockers most commonly used in therapy, mainly as antihypertensives, include Alprenolol (Merck Index, X ed., No. 304), Atenolol (Merck Index, X ed., No. 868), Carteolol (Merck Index, X ed., No. 1850), Metoprolol (Merck Index, X ed., No. 6027), Nadolol (Merck Index, X ed., No. 6195), Oxprenol (Merck Index, X ed., No. 6820), Pindolol (Merck Index, X ed., No. 7317), Propanolol (Merck Index, X ed., No. 7740), and Timolol (Merck Index, X ed., No. 9284).

Furthermore, some compounds have been described wherein the 3-aryloxy-2-hydroxy-propanamine moiety of beta-blockers is linked at different positions of the 4-aryl-dihydropyridine moiety endowed with calcium-antagonist activity.

Baldwin J.J. et al., J. Med. Chem., 1981, 24 628-631 disclose compounds wherein the beta-blocker chain is linked to the aryl moiety of 4-aryl-dihydropyridine at position 4, but these compounds resulted scarcely active; European Patent Application No. 179386 (Bayer A.G.) describes compounds wherein the beta-blocker chain is linked at the carboxy group at position 3 of the 4-aryl-dihydropyridine.

Now we have found a novel class of compounds wherein both a typical beta-blocker structure and a calcium-antagonist moiety are suitably interconnected and show a remarkable antihypertensive activity.

It is therefore an object of this invention to provide a compound of formula:

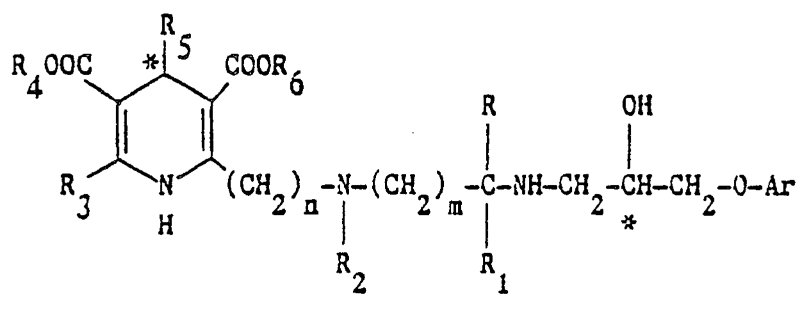

(I)

wherein

Ar is a mono- or dicyclo aromatic or heteroaromatic ring system having from 5 to 10 atoms in the aromatic nucleus optionally substituted by one or more substituents selected from halogen, hydroxy, $C_1$-$C_5$ alkyl, $C_2$-$C_5$ alkenyl, $C_1$-$C_3$ alkoxy, alkoxyalkyl having from 1 to 5 carbon atoms both in the alkyl and in the alkoxy moiety and an optional unsaturation in the chain, phenoxy, phenylalkoxy, aminocarbonylalkyl having from 1 to 3 carbon atoms in the alkyl moiety, cyano, carboxy, carbamoylalkyl, aminocarboxy, amino, mono- and dialkylamino where the nitrogen atom may be a part of a ring;

R and $R_1$, the same or different, are hydrogen or $C_1$-$C_3$ alkyl;
$R_2$ is a straight or branched $C_1$-$C_7$ alkyl or a straight or branched $C_2$-$C_7$ alkenyl optionally substituted by aryl, additionally $R_2$ may be X-$R_7$ where X is CO, CS or $SO_2$ and $R_7$ is an alkyl or an alkoxyalkyl having from 1 to 5 carbon atoms both in the alkyl and the alkoxy moiety, hydroxy, $C_1$-$C_3$ alkoxy, mono- or di-alkylamino having from 1 to 5 carbon atoms in the alkyl moiety, or $C_1$-$C_5$ alkylthio;
$R_3$ is cyano, amino or $C_1$-$C_3$ alkyl optionally substituted by fluorine atoms;
$R_4$ and $R_6$, the same or different, are alkyl or alkoxyalkyl having from 1 to 5 carbon atoms both in the alkyl and alkoxy moiety;
$R_5$ is a ring selected from phenyl, naphthyl, tetrahydronaphthyl and indanyl wherein said ring may be substituted by one or more substituents selected from halogen, hydroxy, alkyl, alkenyl, alkoxy, alkenyloxy, alkoxyalkyl, alkanoyl, trifluoromethyl, amino, nitro, carbamoyl, cyano, alkylthio, carbamoylalkyl and alkanoylamino having up to 6 carbon atoms in the alkyl moiety;
m and n, the same or different, are 1, 2 or 3;
and the salts thereof with organic or inorganic pharmaceutically acceptable acids.

The compound of formula I have at least two asymmetric carbon atoms and can therefore exist as stereoisomers.

Another object of this invention is to provide the compounds of formula I both as stereoisomeric mixture and as single stereoisomers.

The single stereoisomers are obtained by stereoselective synthesis or by separation from the stereoisomeric mixture according to known techniques as fractional crystallization, chromatography and resolution by means of salification or preparation of derivatives with optically active compounds.

The salts of the compounds of this invention with pharmaceutically acceptable organic and inorganic acids are prepared according to conventional methods.

Examples of pharmaceutically acceptable acids are hydrochloric, hydrobromic, phosphoric, sulfuric, lactic, succinic, tartaric, acetic, salicylic, citric, benzoic, p-hydroxybenzoic, naphthalene-2-sulfonic, adipic and pimelic acid.

The compounds of formula I and their pharmaceutically acceptable salts are active on the cardiovascular system as antivasospastics, antiangina agents, antihypertensives and vasodilators.

Unless otherwise stated, when used in connection with R, $R_1$, $R_2$, $R_3$, $R_4$, $R_5$, $R_6$ ,$R_7$ and Ar, alkyl means a straight or branched alkyl having preferably from 1 to 6 carbon atoms (typical examples being methyl, ethyl, n-propyl, 1-propyl, n-butyl, sec-butyl, ter-butyl, n-pentyl and n-hexyl); the alkyl moiety of alkoxy and alkoxyalkyl is intended to have the meaning cited above for alkyl; aryl and aromatic or heteroaromatic system mean an aromatic or heteroaromatic nucleus having 5 (heteroaromatic), 6, 9, 10 or 12 members and the heteroaromatic nucleus may have from 1 to 3 heteroatoms selected from oxygen, sulfur and nitrogen (typical examples being furyl, pyrrolyl, isoxazolyl, 1,2,5-triazolyl, oxazolyl, phenyl, pyridyl, indenyl, indolyl, naphthyl, benzofuranyl, isobenzofuranyl, quinolyl, diphenyl and phenylipyridyl); alkenyl means a branched or straight alkenyl radical having 2 to 7 carbon atoms and at least one unsaturation (typical examples being vinyl, allyl, 2,2-dimethylvinyl, and 1-butadienyl); alkanoyl means a residue of a straight or branched carboxylic acid having 1 to 5 carbon atoms (typical examples being formyl, acetyl, butyroyl and isobutyroyl).

A preferred group of compounds of formula I includes those compounds wherein Ar has the same meaning as the aryl group in known beta-blockers.

Preferred meanings of Ar comprise phenyl, naphthyl, isobenzofuranyl, benzofuranyl, 3,4-dihydrocarbostyryl, benzopyranyl, tetrahydronaphthyl, carbazolyl, indenyl, indolyl and 1,2,5-thiadiazolyl, optionally substituted.

Examples of possible substituents comprise one or more fluorine, chlorine, bromine, acetyl, allyl, carbamoylmethyl, butyroylamino, cyclohexyl, cyano, hydroxy, butyroyl, acetylamino, methoxycarbonyl, methoxyethyl, methoxy, allyloxy, cyclopentyl, cyclopropyl, morpholine, ethyl and isobutyroyl.

Specific examples of preferred substituted aryls comprise 2-methoxyphenyl, 2-allyloxyphenyl, 2-cyanophenyl, 2-methylphenyl, 2-allylphenyl, 4-carbamoylmethylphenyl, 4-hydroxyphenyl, 4-morpholino-1,2,5-thiadiazol-3-yl, indol-4-yl and 3,4-dihydro-1(H)-carbostiryl-5-yl.

Another group of preferred compounds includes those compounds wherein $R_5$ is a substituted phenyl and more particularly 3-nitrophenyl, 2-nitrophenyl, 2-chlorophenyl, 2-trifluoromethyl-phenyl, and 2,3-dichlorophenyl.

A third group of preferred compounds includes those compounds wherein R and $R_1$, the same or different, are hydrogen or methyl.

A fourth group of preferred compounds comprises those compounds wherein $R_3$ is methyl or trifluoromethyl.

Finally, among the compounds of general formula I a group of most preferred compounds comprises the compounds wherein

Ar is selected from 2-methoxyphenyl, 2-allyloxyphenyl, 2-cyanophenyl, 2-methylphenyl, 2-allylphenyl, 4-carbamoylmethylphenyl, 4-hydroxyphenyl, 4-morpholino-1,2,5-thiadiazol-3-yl, indol-4-yl and 3,4-dihydro-1-(H)-carbostiryl-5-yl;

R and $R_1$, the same or different, are hydrogen or methyl;

$R_2$ has the meanings mentioned above in connection with formula I;

$R_3$ is methyl or trifluoromethyl;

$R_4$ and $R_6$ have the meanings mentioned above in connection with formula I;

$R_5$ is phenyl substituted by one or more substituents selected from nitro, trifluoromethyl and halogen;

X is a carbonyl or sulfonic group.

$R_7$ is methyl, ethyl, isopropyl, phenyl, dimetylamino, diethylamino, methoxy or ethoxy.

A further object of this invention is to provide a process for preparing a compound of formula I comprising (i) the condensation of the $R_2$-NH- group of a diamine of formula

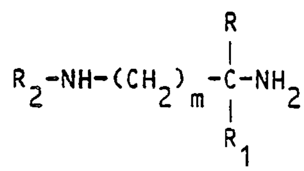

(II)

wherein R, $R_1$, and m have the meanings mentioned above in connection with formula I and $R_2$ is a member selected from alkyl and alkenyl wherein both members, straight or branched, have from 1 to 7 carbon atoms and are optionally substituted by an aryl group;

with a 2-alkylen-4-aryl-dihydropyridine and (ii) the introduction of a 3-aryloxy-2-hydroxy-propyl radical on the primary amino group of (II).

Both steps can be performed according to several procedures and step (ii) may also precede step (i).

In the following description of the synthesis method R, $R_1$, $R_3$, $R_4$, $R_5$, $R_6$, Ar, m and n have the meanings already mentioned above in relation to formula I, R2 has the meanings already mentioned above in relation to formula II.

Before performing step (i), the primary amino group of diamine II is preferably protected according to conventional techniques. Examples of suitable protective groups comprise benzyl, benzyloxycarbonyl, ter.butyloxycarbonyl and phthalimido group.

Step (i) affords, after condensation of a 2-akylen-4-aryldihydropyridine compound with a protected diamine (II) and subsequent removal of the protective group, a compound of formula

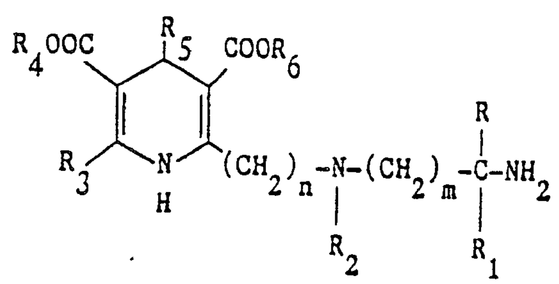

(III)

Step (ii) involves condensation of the compound (III) with an epoxide (IV) or with a compound of formula or (V)

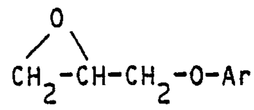

(IV)

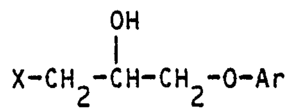

(V)

wherein X is halogen, alkyl or arylsulfonyloxy, to afford a compound of formula I.

A preferred way of performing step (i) comprises the condensation of a protected diamine (II) with a 2-alkylen-4-aryl-dihydropyridine compound of formula

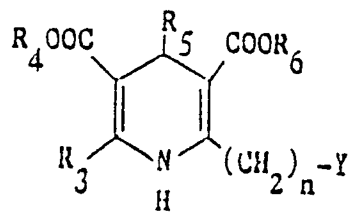

(VI)

wherein Y is a leaving group.

Examples of preferred leaving groups comprise chlorine, bromine, iodine, alkylsulfonyloxy, and arylsulfonyloxy. Typical example of a preferred leaving groups are methanesulfonyloxy, benzenesulfonyloxy and p-toluensulfonyloxy.

The condensation of compound (VI) with a protected diamine (II) is performed in an inert solvent and in the presence of a base.

Another preferred way of performing step (i) comprises the condensation of a protected diamine (II) with a 2-alkylen-4-aryl-dihydropyridine compound of formula

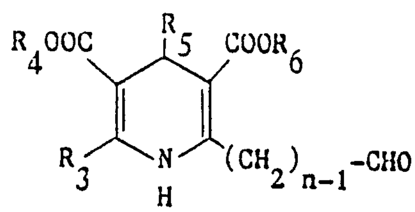

(VII)

The condensation of compound (VII) with a protected diamine (II) is performed in an inert solvent, optionally in the presence of a dehydrating agent, and subsequent reduction. This reduction is preferably carried out by catalytic hydrogenolysis or with reducing agents such as sodium boron hydride and sodium cyano hydride.

When step (ii) is carried out before step (i), the diamine (II) is optionally protected on the secondary amino group ($R_2$-NH-), the optionally protected diamine (II) is then reacted with a compound of formula (IV) or (V) to give, after removal of the possible protective group, a compound of formula

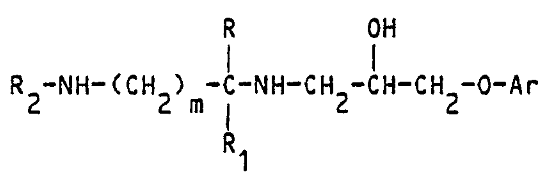

(VIII)

and finally the compound (VIII) is reacted with a compound of formula (VI) or (VII).

Protection and removal of the protective group are carried out as described above.

The condensation reaction of the optionally protected diamine (II) with a compound of formula (IV) or

(V) is also performed as described above in connection with the reaction of compound (III) with compounds (IV) or (V).

Even the reaction of compound (VIII) with compound (VI) or (VII) is carried out as described above.

Another way for performing step (ii) before step (i) comprises preparing a compound of formula

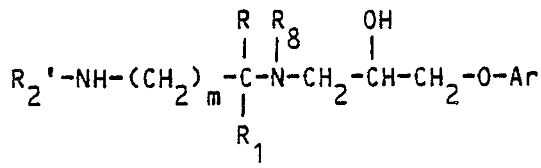
(IX)

wherein
$R_2'$ is hydrogen or a member selected from alkyl and alkenyl wherein both members, straight or branched, have from 1 to 7 carbon atoms and are optionally substituted by an aryl group; and
$R_8$ is a protective group;
by reacting an amide of formula

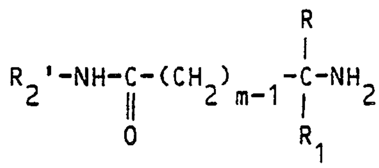
(X)

with a compound of formula (IV) or (V) to give a compound of formula

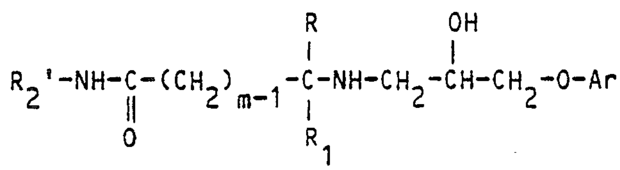
(XI)

which is then protected by a protective agent and reduced to afford a compound of formula (IX).

Preferred meaning of $R_8$ is trifluoromethyl.

Reduction is preferably carried out with hydrides such as lithium aluminum hydride, lithium boron hydride, cobalt boron hydride and borane.

Another way of performing step (i) comprises the reaction of a compound of formula (IX) with a compound of formula (VI) or (VII) to give a compound of formula

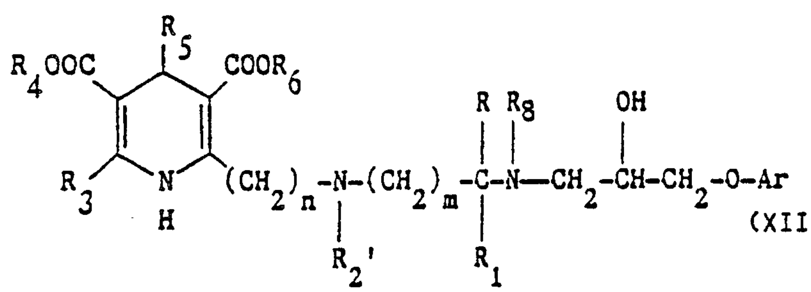
(XII)

This reaction is carried out as described above in relation to the preparation of compound (III).

When $R_2'$ is a member selected from alkyl and alkenyl wherein both members, straight or branched, have from 1 to 7 carbon atoms and are optionally substituted by an aryl group, the compound of formula I can be easily prepared by simple removal of the protective group from compound (XII) according to conventional techniques such as, for example, a treatment with an acid.

When $R'_2$ is hydrogen, the compound of formula I is prepared by alkylation or acylation of compound (XII) and subsequent removal of the protective group.

The alkylation is carried out in an inert solvent and in the presence of a base with an alkylating agent of formula

$R_2$-X′ (XIII)

wherein X′ is chlorine or bromine;
or with methyl sulfate.

Reacting a compound of formula (XII), wherein $R_2'$ is hydrogen, with suitable reactive derivatives of a carboxylic or a sulfonic acids, such as chlorides and anhydrides, or of carbonic acids, such as chlorocarbonates and chlorocarbamates; with isocyanates or isothiocyanates and subsequent removal of the protective group according to conventional methods, the compounds of formula I wherein $R_2$ is X-$R_7$ are obtained.

The condensation reaction is carried out in a suitable inert solvent, optionally in the presence of an organic or inorganic base as acid acceptor, at a temperature of from 0°C to the reflux temperature of the reaction mixture.

Examples of suitable solvents comprise hydrocarbons such as toluene and cyclohexane, ethers such as tetrahydrofuran and dioxane, chlorinated hydrocarbons such as dichloromethane, chloroform and tetrachloroethylene.

Examples of suitable bases comprise inorganic bases such as alkali and alkaline-earth metals hydroxydes, bicarbonates and carbonates and organic bases such as triethylamine and pyridine, which can also act as a solvent. Preferred bases are sodium and potassium hydroxydes, bicarbonates and carbonates.

The process of this invention contemplate also the protection of the amino group via formation of a heterocyclic ring such as the conversion of compound (XI) to an oxazolidine compound of formula

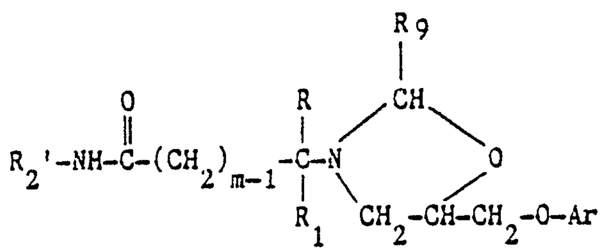

(XIV)

wherein $R_9$ is $C_1$-$C_6$ alkyl or phenyl.

Compounds of formula (XIV) are prepared by reacting a compound (XI) wherein the nitrogen atom linked to $R_2'$ is protected or a compound (XI) where $R_2'$ is $R_2$ ,with an aldehyde of formula

$R_9$-CHO (XV)

At the end of the process, the oxazolidine ring is opened by treatment with acids and the hydroxy group is thus restored.

The compounds of formula I of this invention have at least two asymmetry centers which are marked by an asterisk.

The asymmetric synthesis for preparing diastereoselectively the compounds of formula I can be performed, for example, via optically active intermediate compounds of formula (III), (IV), (V), (VI), (VII) or (IX).

The compounds of formula (VI) and (VII) are known, for example, from European Patent Applications 169 009 and 212 340 or can be easily prepared according to Hantszch method by reacting the compounds of formula $R_5$-CHO (XVI) and $R_3$-C($NH_2$)=CH-COO$R_4$ (XVII) and a keto ester of formula $R_6$OOC-$CH_2$-CO-$(CH_2)_{n-1}$-$R_{10}$ (XVIII) wherein $R_{10}$ is -$CH_2$-Y or -CHO or any other similar group such as -$CH_2$OH.

The compounds of formula I compounds proved to be useful in the treatment of cardiovascular pathologies as antivasospastics, antiangina agents, antihypertensives and vasodilators.

The pharmacological actity of the compound of this invention has been tested both in vitro and in vivo - (Example 16).

The compound of formula I are endowed with a remarkable calccium-antagonist and beta blocking action and are useful in producing antihypertensive effects with concomitant reduction of heart rate, contrary to known calcium-antagonists in which the hypotensive effect is accompained by tachycardia.

In addition to a remarkable action on cardiovascular system, the compounds of formula I show interesting properties which make them particularly suitable for use in the pharmaceutical field.

Contrary to the known drugs having a dihydropyririne structure, the compounds of formula I have a considerable chemical and physical stability.

A still further object of the present invention is to provide a pharmaceutical composition containing a

compound of formula I or a pharmaceutically acceptable salt thereof together with one or more solid or liquid, organic or inorganic pharmaceutical excipients, such as diluents, preservatives, humectants, dyes, flavours and the like.

The pharmaceutical compositions of the present invention may be administered as solid dosage forms, such as tablets, pills, capsules, granules and suppositories, or as liquid dosage forms such as syrups, suspensions, emulsions and solutions suitable for oral or parenteral administration, or also as dosage forms suitable for transdermal administration such as wax plasters.

The compounds of the present invention may also be compounded in slow releasing dosage forms.

The preparation of the pharmaceutical compositions of the present invention is carried out according to conventional techniques.

The doses of the compound of formula I will vary according to several factors such as the specific activity of the single compound of formula I, the therapy and the individual patient response as well as the selected administration route.

Generally the amount of the compound of formula I to be administered will be in the range of from 0.1 mg to 200 mg per day in one or more repeated doses.

For the purpose of better illustrating the present invention, the following examples are now given.

## Example 1

### Preparation of ethyl 1,4-dihydro-6-methyl-2-(N-methyl-N-(2-(2-hydroxy-3-(2-methoxyphenoxy)-propylamino)-2-methylpropyl)aminomethyl)-4-(3-nitrophenyl)-3,5-pyridinedicarboxylate

### (COMPOUND 1)

A mixture of ethyl 1,4-dihydro-2-formyl-6-methyl-4-(3-nitrophenyl)-3,5-pyridinedicarboxylate (5.0 g; 13 mmoles), and of N-methyl-2-(2-hydroxy-3-(2-methoxyphenoxy)-propylamino)-2-methylpropylamine (3.65 g; 13 mmoles) in 95% ethanol (70 ml) is stirred for one hour at room temperature.

After cooling to 0-5 °C, water (31.8 ml), acetic acid (10.9 ml), sodium acetate (3.45 g) and finally sodium boron hydride (2.55 g; 70 mmoles) are added. The mixture is left for four hours at room temperature. After solvent evaporation, water is added to the residue which is basified with dilute ammonia and extracted with methylene chlorie.

The organic phase is dried over sodium sulfate and after evaporation of the solvent the residue is purified by chromatography on a silica gel column (230-400 mesh), eluting with methylene chloride and increasing amounts of ethanol up to a 95:5 ratio.

Ethyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarboxylate is obtained as 1:1 mixture of the two diastereomer pairs (thin layer chromatography - eluent ethyl acetate:$NH_4OH$ = 99.1).

The two diastereomeric pairs had Rf = 0.22 and Rf = 0.17, respectively.

$^1$H-NMR diastereomeric mixture - free base (300 MHz, $CDCl_3$) delta (ppm); 1.18 (6H, 4s); 1,22 (6H, 2t); 2.40 (6H, s); 2.59 (2H, m); 3.84 (3H, 2s); 3.93 (2H, m); 5.09 (1H, s); 7.35 (1H, 2t); 7.62 (1H, d); 7.98 (1H, d); 8.12 (1H, bs).

The signal at 7.35 ppm is splitted because of the presence of two diastereoisomers and become simple in the spectra of the two pure diastereooisomers.

The two diastereoisomers were separated by chromatography on a silica gel column (230-400 mesh) eluting with ethylacetate: $NH_4OH$ = 99:1.

The less polar product is salified with a solution of hydrochloric acid in ether to give a crystalline hydrochloride salt melting at 116-118 °C (ethyl acetate).

## Example 2

Preparation of methyl 1,4-dihydro-6-methyl-4-(3-nitrophenyl)-2-(2-oxo-ethyl)-3,5-pyridinedicarboxilate

A solution of methyl 1,4-dihydro-2,6-dimethyl-4-(3-nitrophenyl)-3,5-pyridinedicarboxylate (34.6 g; 0.10 mmoles) and N,N-dimethylformamide dimethylacetale (14.7 g; 0.12 mmoles) in dimethylformamide is heated to reflux under nitrogen atmosphere for 16 hours.

The solvent is evaporated and the crude product is extracted with toluene and water. The organic phase is separated and, after drying over sodium sulfate, the solvent is evaporated.

Methyl 1,4-dihydro-2-(2-(N,N-dimethylamino-ethenyl)-6-methyl-4-(3-nitrophenyl)-3,5-pyridinedicarboxylate is obtained which is directly hydrolized without any further purification.

A solution of this compound (6 g; 15 mmoles) in acetone (60 ml) is treated for one hour under nitrogen atmosphere and at room temperature with hydrochloric acid 6N (6.96 ml).

The cold solution is evaporated and some water is added; the solution is filtered, the filtrate is diluted with water and extracted with methylene chloride.

After drying over sodium sulfate, the solvent is evaporated obtaining methyl 1,4-dihydro-6-methyl-4-(3-nitrophenyl)-2-(2-oxo-ethyl)-3,5-pyridinedicarboxilate, chromatographically pure oil (thin layer chromatography - eluent methylene chloride : methanol:$NH_4OH$ = 94.5:5:0.5).

Mass spectrum (chemical ionization, positive ions, isobutane): m/e 374 $(M+1)^+$.

Example 3

Preparation of methyl 1,4-dihydro-6-methyl-2-(N-methyl-N-(2-(2-hydroxy-3-(2-methoxyphenoxy)propylamino)-2-methylpropyl)-aminoethyl)-4-(3-nitrophenyl)-3,5-pyridinedicarboxylate

Operating in a way similar to that described in example 1 but using 1,4-dihydro-6-methyl-4-(3-nitrophenyl)-2-(2-oxo-ethyl)-3,5-pyridinedicarboxylate as the starting aldehyde and carrying out the reduction reaction in dioxane for 12 hours methyl 1,4-dihydro-6-methyl-2-(N-methyl-N-(2-(2-hydroxy-3-(2-methoxyphenoxy)-propylamino)-2-methylpropyl)-aminoethyl)-4-(3-nitrophenyl)-3,5-pyridinedicarboxylate is obtained, yellow chromatographycally pure oil (thin layer chromatography -eluent methylene chloride:methanol:$NH_4OH$ = 94.5:5:0.5.

$^1$H-NMR (300 MHz, $CDCl_3$ ): delta (ppm): 1.20 (6H, 4s); 2.34 (3H, 2s); 2.44 (3H, 2s); 2.52 (2H, bd); 3.62 (6H, m); 3.83 (3H, s); 4.05 (2H, m); 4.25 (1H, m); 5.06 (1H, s); 7.35 (1H, 2t); 7.61 (1H, bd); 7.95 (1H, dd); 8.10 (1H, bs).

Working in a similar way, from the levo rotatory enantiomer of N-methyl-2-(2-hydroxy-3-(2-methoxyphenoxy)-propylamino)-2-methylpropylamine (prepared as described in example 8) methyl 1,4-dihydro-6-methyl-2-(N-methyl-N-(2-(2-hydroxy-3-(2-methoxyphenoxy)-propylamino)-2-methylpropyl)-aminoethyl)-4-(3-nitrophenyl)-3,5-pyridinedicarboxylate is obtained as a mixture of two diastereoisomers.

Example 4

Preparation of N-methyl-3-amino-3-methyl-butyramide

A mixture of methyl 3-amino-3-methyl-butyrate hydrochloride (10.5 g ;62.6 mmoles) and methyl amine (33% solution in ethanol; 55ml) is refluxed for 18 hours. The solvent is evaporated, the crude is dissolved in methylene chloride (100 ml) and washed with a saturate aqueous solution of sodium chloride (10 ml). After drying over sodium sulphate and evaporation of the solvent to dryness, N-methyl-3-amino-3-methyl-butyramide is obtained; chromatographically pure colorless oil (T.L.C., eluent - methylene chloride : methanol : $NH_4OH$ = 80 : 15 : 1)

Mass spectrum (chemical ionization, positive ions, isobutane) : m/e 131 $(M+1)^+$; 114.

$^1$H-NMR (60 MHz, $CDCl_3$ ): delta (ppm):1.2 (6H, s); 2.2 (2H,s); 2.8 (3H, s).

Example 5

Preparation of (-)-N-methyl-2-(2-hydroxy-3-(2-methoxy-phenoxy)-propylamino)-2-methyl-propionamide

a) A mixture of 1,2-epoxy-3-(2-methoxyphenoxy)-propane (14 g; 0.077 moles) and N-methyl-2-amino-2-methylpropionamide (9.0 g; 0.077 moles) is absolute ethanol (90 ml) is heated at 80 °C for two hours.

After evaporation of the solvent, the crude product is dissolved in ethyl acetate (100 ml) and a solution of ethyl acetate/concentrate HCl 85:15 is added dropwise, while cooling with ice, till pH is substantially acid.

The oily phase which precipitates by acidification is separated and dissolved in water (100 ml).

The so obtained solution is brought to pH 5-6 with a dilute sodium hydroxide solution and is extracted twice with ethyl acetate (50 ml). The aqueous phase is separated and made alkaline with potassium bicarbonate; the solution is then saturated with sodium chloride and extracted with ethyl acetate (3x80 ml).

The organic phase is dried over sodium sulfate and filtered and the solvent is evaporated. N-methyl-2-(2-hydroxy-3-(2-methoxyphenoxy)-propylamino)-2-methyl-propionamide is thus obtained as a chromatographycally pure colourless oil (thin layer chromatography - eluent, chloroform:methanol:$NH_4OH$ = 79:15:1).
$^1$H-NMR (300 MHz, $CDCl_3$): delta (ppm): 1.35 (6H, s); 1.70 (2H, bs; exchange with $D_2O$); 2.72 (2H, m); 2.78 (3H, d); 3.85 (3H, s); 3.98 (1H, dd); 4.05 (1H, m); 4.15 (1H, dd); 6.90-7.02 (4H, m); 7.35 (1H, bq).

Mass spectrum (chemical ionization, positive ions, isobutane) : m/e 297 $(M+1)^+$; 238.

b) A mixture of N-methyl-2-(2-hydroxy-3-(2-methoxyphenoxy)-propylamino)-2-methyl-propionamide (146 g; 0.49 moles) and (+)-camphosulfonic acid (114 g; 0.49 moles) in ethyl acetate (1.5 l) is heated to reflux until completely dissolved.

The solution is cooled to room temperature and after 10 hours is filtered.

The crystalline product which separates is washed with little ethyl acetate and recrystallized three times from isopropyl alcohol to obtain (-)-N-methyl-2-(2-hydroxy-3-(2- methoxyphenoxy)-propylamino)-2-methyl-propionamide camphosulfonate as a crystalline white solid melting at 154-156 °C, $(alpha)_D = +12.25°$ (c = 5%, ethanol).

A solution of this salt is made alkaline with potassium carbonate and extracted with methylene chloride to give the corresponding free base in the form of a colourless oil.

Operating in a similar way, the following compounds were prepared :

N-isopropyl-2-(2-hydroxy-3-(2-methoxyphenoxy)-propylamino)-2-methylpropionamide

Mass spectrum (chemical ionization, positive ions, isobutane) : m/e 325 $(M+1)^+$; 238
$^1$H-NMR (300 MHz, $CDCl_3$): delta (ppm): 1.38 (6H, d); 1.58 (6H, s); 2.96 (2H, m); 4.12 (3H, s); 4.21-4.38 (4H); 7.14-7.28 (4H).

N-butyl-2-(2-hydroxy-3-(2-methoxyphenoxy)-propylamino-2-methyl-propionamide

Mass spectrum (chemical ionization, positive ions, isobutane) : m/e 339 $(M+1)^+$; 238.
$^1$H-NMR (300 MHz, $CDCl_3$): (ppm): 1.15 (3H, t); 1.58 (6H, s); 1.48-1.75 (4H); 2.96 (2H, m); 3.45 (2H, q); 4.11 (3H, s); 4.20-4.63 (3H); 7.13-7.28 (4H).

2-(2-hydroxy-3-(2-methoxyphenoxy)-propylamino)-2-methyl-propionamide

Mass spectrum (chemical ionization, positive ions, isobutane): m/e 283 $(M+1)^+$ ; 238.
$^1$H-NMR (300 MHz, $CDCl_3$): delta (ppm): 1.35 (6H, s); 2.78 (2H, m); 3.85 (3H, s); 3.98 (1H, dd); 4.05 (1H, m); 4.10 (1H, dd); 6.88-7.02 (4H, m).

N-methyl-3-(2-hydroxy-3-(2-methoxyphenoxy)-propylamino-3-methyl-butyramide

Mass spectrum (chemical ionization, positive ions, $NH_4OH$): m/e 311 $(M+1)^+$; 143.
$^1$H-NMR (300 MHz), $CDCL_3$ : delta (ppm): 1.14 and 1.15 (6H, 2s); 2.26 (2H, s); 2.73 (3H, d), 2.75 (2H, m),

3.75 (3H, s); 3.70-4.10 (3H); 6.86-7.03 (4H).

N-methyl-2-(2-hydroxy-3-(1-naphthoxy)-propylamino)-2-methylpropionamide

Mass spectrum (chemical ionization, positive ions, $NH_4OH$) m/e 317 $(M+1)^+$; 258.
$^1$H-NMR (300 Mhz, $CDCl_3$ ): delta (ppm); 1.32 and 1.33 (6H, 2s); 2.65 (3H, d); 2.78 (1H, dd), 2.86 (1H, dd); 4.15-4.24 (3H); 6.80 (1H, d); 7.36 (1H, t); 7.42-7.52 (3H); 7.80 (1H, bd); 8.20 (1H, bd).

N-methyl-2-(2-hydroxy-3-(4-carbazolyloxy)-propylamino)-2-methyl-prpionamide

Mass spectrum (chemical ionization, positive ions, $NH_4OH$) m/e 356 $(M+1)^+$; 297.
$^1$H-NMR (300 MHz, $CDCl_3$ ): delta (ppm): 1.35 (6H, s); 2.68 and 2.70 (3H, 2s), 2.78-2.94 (2H); 4.20-4.30 (3H); 6.67 (1H, d); 7.08 (1H, d); 7.18-7.44 (4H); 8.21 (1H, d)

The above mentioned compounds have been obtained in the form of chromatographically pure oils (T.L.C.; eluent, methylene chloride : methanol : $NH_4OH$ = 94.5 : 5 : 0.5)

## Example 6

Preparation of (-)-N-methyl-2-((2-hydroxy-3-(2-methoxyphenoxy)-propyl)-triphenylmethylamino)-2-methylpropionamide

A mixture of (-)-N-methyl-2-(2-hydroxy-3-(2-methoxyphenoxy)-propylamino)-2-methyl-propionamide (18.0 g; 0.061 moles), prepared as disclosed in example 4, triphenylmethyl chloride (17.5 g; 0.061 moles) and triethylamine (12 ml; 0.085 moles) in toluene (180 ml) is refluxed under stirring for 10 hours. After cooling to room temperature, the reaction mixture is washed with water (100 ml), with a potassium bicarbonate aqueous solution (80 ml) and finally with water (80 ml) again.

The organic phase is dried over sodium sulfate and filtered. The solid crude product obtained after evaporation of the solvent, is purified by chromatography on silica gel column (230-400 mesh) eluting with methylene chloride. (-)-N-methyl-2-((2-hydroxy-3-(2-methoxyphenoxy)-propyl)-triphenylmethylamino)-2-methylpropionamide is obtained as a chromatographycally pure oil (thin layer chromatography -eluent, ethyl acetate:gasoline = 8:2).
$(alpha)_D$ = -40.2° (c=5%, ethanol).
$^1$H-NMR (300 MHz, $CDCl_3$): delta (ppm); 1.14 (3H, s); 1.22 (3H, s); 2.33 (1H, dd); 2.48 (3H, d); 2.68 (1H, dd); 3.55 (1H, dd); 3.76 (3H, s); 4.00-4.10 (2H; 6.62 (1H, bd); 6.80-6.95 (3H, m); 7.20-7.35 (9H, m); 7.50 (6H, bd).

Operating in a similar way, the following compounds were prepared:

N-isopropyl-2-((2-hydroxy-3-(2-methoxyphenoxy)-propyl)-triphenylmethylamino-2-methylpropionamide

Chromatographically pure oil (thin layer chromatography -eluent, methylene chloride : methanol:$NH_4OH$ = 98:4:0.2

Mass spectrum (chemical ionization, positive ions, ammonium hydroxide): m/e 568 $(M+2)^+$; 325; 243.
$^1$H-NMR (300 MHz, $CDCl_3$ ): delta (ppm): 0.89 (3H, d); 0.92 (3H, d); 1.10 (3H, s); 1.21 (3H, s); 2.42 (1H, dd); 2.57 (1H, dd); 3.64 (1H, dd); 3.75 (3H, s); 3.87 (1H, m); 3.99 (1H, dd); 4.03 (1H, m); 6.63 (1H, bd); 6.80-6.94 (3H); 7.20-7.32 (9H, m); 7.52 (6H, bd).

N-butyl-2-((2-hydroxy-3-(2-methoxyphenoxy)-propyl)-triphenylmethylamino)-2-methylpropionamide

Chromatographically pure oil (thin layer chromatography -eluent, methylene chloride:methanol:$NH_4OH$ = 160:15/1)

Mass spectrum (chemical ionization, positive ions, ammonium hydroxide): m/e 581 $(M+1)^+$; 339; 243.
$^1$H-NMR (300 MHz, $CDCl_3$): delta (ppm): 0.78 (3H, t); 1.12 (3H, s); 1.22 (3H, s); 1.08-1.25 (4H); 2.37 (1H,

dd); 2.58 (1H, dd); 2.85 (1H, m); 3.05 (1H, m); 3.59 (1H, dd); 3.76 (3H, s); 4.01 (1H, dd); 4.01 (1H, m); 6.63 (1H, bd); 6.80-6.95 (3H); 7.20-7.35 (9H); 7.50 (6H, bd).

2-((2-hydroxy-3-(2-methoxyphenoxy)propyltriphenylmethylamino)-2-methylpropionamide

Chromatographicaaly pure oil (thin layer chromatography -eluent, methylene chloride:methanol:$NH_4OH$ = 160:15:1)

Mass spectrum (chemical ionization, positive ions, ammonium hydroxide): m/e 525 $(M+1)^+$; 480; 243;115.

$^1$H-NMR (300 Mhz, $CDCl_3$ ): delta (ppm); 1.16 (3H, s); 1.25 (3H, s); 2.41 (1H, dd); 2.60 (1H, dd); 3.61 (1H, dd); 4.01 (1H, dd); 4.04 (1H, m); 6.58 (1H, bd); 6.80-6.94 (3H, m); 7.22-7.35 (9H, m); 7.50 (6H, bd).

## Example 7

Preparation of (-)-N-(2-((2-hydroxy-3-(2-methoxyphenoxy)-propyl)-triphenylmethylamino)-2-methylpropyl)-methylamine

A solution of (-)-N-methyl-2-((2-hydroxy-3-(2-methoxyphenoxy)-propyl)-triphenylmethylamino)-2-methylpropionamide (25.1 g; 0.047 moles), prepared as described in example 6, in tetrahydrofuran (200 ml) is added, dropwise under stirring and in a inert atmosphere, to a suspension of lithium aluminium hydride (3.55 g; 0.093 moles) in anhydrous tetrahydrofuran (50 ml).

On completion of the addition, the reaction mixture is heated to reflux for two hours.

After cooling on a ice bath, the lithium aluminium hydride excess is decomposed by adding in succession dropwise a solution of tetrahydrofuran/water 9:1 (5 ml), and then water (4 ml), 15% sodium hydroxide (4 ml), and again water (10 ml).

The precipitate is filtered and washed with ethyl ether; the organic phase is dried over sodium sulfate and filtered. (-)-N-(2-((2-hydroxy-3-(2-methoxyphenoxy)-propyl)-triphenylmethylamino)-2-methylpropyl)-methylamine is obtained by solvent evaporation .

Mass spectrum (chemical ionization, positive ions, ammonium hydroxide): m/e 526 $(M+2)^+$; 243.

$^1$H-NMR (300 MHz, $CDCl_3$ ): delta (ppm): 0.92 (3H, s); 1.00 (3H, s); 2.30 (3H, s); 2.34 (2H, s); 2.35-2.46 (2H, m); 3.68 (1H, dd); 3.80 (3H, s); 4.01 (1H, dd); 4.05 (1H, m); 6.68 (1H, bd); 6.80-6.90 (3H, m); 7.20-7.34 (9H, m); 7.52 (6H, bd).

Working in a similar way the following compounds were prepared:

N-(2-((2-hydroxy-3-(2-methoxyphenoxy)-propyltriphenylmethylamino)-2-methylpropyl)-isopropylamine

chromatographycally pure oil (thin layer chromatography -eluent, $CH_2Cl_2$:methanol:$NH_4OH$ = 98:4:0.2)

Mass spectrum (chemical ionization, positive ions, ammonium hydroxide): m/e 554 $(M+2)^+$; 243.

$^1$H-NMR (300 MHz, $CDCl_3$): delta (ppm); 0.88-0.95 (12H); 2.32 (2H, s); 2.45 (2H, bd); 2.60 (1H, m); 3.62 (1H, ddd); 3.79 (3H, s); 3.94 (1H, dd); 3.98 (1H, m); 6.64 (1H, d); 6.83 (3H, m), 7.25 (9H, m); 7.52 (6H, bd).

N-(2-((2-hydroxy-3-(2-methoxyphenoxy)-propyltriphenylmethylamino)-2-methylpropyl)butylamine

chromatographycally pure oil (thin layer chromatography -eluent, $CH_2Cl_2$:methanol:$NH_4OH$ = 98:4:0.2

Mass spectrum (chemical ionization, positive ion, ammonium hydroxide): m/e 568 $(M+2)^+$; 243

$^1$H-NMR (300 MHz, $CDCl_3$): delta (ppm): 0.83 (3H, t); 0.92 (3H, s); 0.98 (3H, s); 1.20 (2H, m); 1.33 (2H, m); 2.37 (2H, s); 2.40-2.52 (4H); 3.65 (1H, dd); 3.80 (3H, s); 3.96 (1H, dd); 3.99 (1H, m); 6.65 (1H, bd); 6.74 (3H, m); 7.26 (9H, m); 7.52 (6H, bd).

2-((2-hydroxy-3-(2-methyoxyphenoxy)-propyltriphenylmethylamino)-2-methylpropyl)-methylpropylamine

chromatographycally pure oil (thin layer chromatography -eluent, $CH_2Cl_2$:methanol:$NH_4OH$ = 160:15:1)

Mass spectrum (chemical ionization, positve ions, ammonium hydroxide): m/e 512 $(M+2)^+$; 480; 243. $^1$H-NMR (300 MHz, $CDCl_3$ ): delta (ppm): 0.85 (3H, s); 0.89 (3H, s); 2.38 (2H, s); 2.42 (2H, d); 3.70 (1H, dd); 3.80 (3H, s); 4.00 (2H, m), 6.65 (1H, dd); 6.80-6.90 (3H, m); 7.20-7.30 (9H, m); 7.50-7.55 (6H, m).

Example 8

Preparation of (-) N-methyl-2-(2-hydroxy-3-(2-methoxyphenoxy)-propylamino)-2-methylpropylamine oxalate

A solution of (-) N-methyl-2-(2-hydroxy-3-(2-methoxyphenoxy)-propylamino)-2-methylpropionamide (50 g ; 0.017 moles), prepared as described in example 4b, and borane methyl sulfite (78.2 ml; 0.82 moles) in tetrahydrofuran (500 ml) is maintained under reflux for 3 hours under nitrogen atmosphere.

The reaction mixture is cooled to 5° C and a solution of concentrate HCl (35 ml) in methanol (200 ml) is slowly added; the mixture is then refluxed for 1 hour. The solvent is evaporated and the crude is collected with water (100 ml). The mixture is made alkaline to pH 8 with dilute ammonia. After addition of potassium bicarbonate till saturation, the mixture is extracted with methylene chloride. The organic phase is dried over sodium sulphate, filtered and evaporated to dryness. The residue is dissolved in hot ethyl acetate (300 ml). The mixture is cooled and a solution of oxalic acid in ethyl acetate is added till acid pH.

After half an hour at room temperature, the crystalline solid is separeted by filtration, washed with ethyl acetate and then with ethyl ether. After drying, (-) N-methyl-2-(2-hydroxy-3-(2-methoxyphenoxy)-methylpropylamino)-2-propylamine oxalate is obtained in the form of a whithe solid melting at 150-152° C.

The base shows an $(alpha)_D$ = -10.8 (water, c = 0.5%)

Working in a similar way the following compounds were prepared:

N-methyl-3-(2-hydroxy-3-(2-methoxyphenoxy)-propylamino)-3-methylbutylamine

White solid, m.p.93° C

Mass spectrum (chemical ionization, positve ions, ammonium hydroxide): m/e 297 $(M+1)^+$;

$^1$H-NMR (300 MHz, $CDCl_3$ ): delta (ppm): 1.11 (6H, s); 1.62 (2H, bt); 2.42 (3H, s); 2.70 (2H, bt); 2.73 (1H, dd), 2.86 (1H, dd); 3.83 (3H, s); 4.01 (1H, m); 4.2 (2H, bs), 6.84-6.95 (4H).

N-methyl-2-(2-hydroxy-3-(1-naphthoxy)-propylamino)-2-methylpropylamine

Chromatographically pure oil (T.L.C.; eluent, methylene chloride : methanol : ammonium hydroxide = 86 : 10 : 0.6)

Mass spectrum (chemical ionization, positve ions, ammonium hydroxide): m/e 303 $(M+1)^+$;258

$^1$H-NMR (300 MHz, $CDCl_3$ ): delta (ppm); 1.07 and 1.08 (6H, 2s); 2.41 (3H, s); 2.47 (2H, d); 2.75 (1H, dd); 2.92 (1H, dd); 4.08-4.21 (3H); 6.82 (1H, d); 7.32-7.50 (4H); 7.79 (1H, m), 8.25 (1H, m).

N-methyl-2-(2-hydroxy-3-(4-carbazolyloxy)-propylamino)-2-methylpropylamine

Chromatographically pure oil (T.L.C.; eluent, methylene chloride : methanol : $NH_4OH$ = 86 : 10 : 0.6)

Mass spectrum (chemical ionization, positve ions, ammonium hydroxide): m/e 342 $(M+1)^+$;29

$^1$H-NMR (300 MHz, $CDCl_3$ ) delta (ppm): 1.09 and 1.11 (6H, 2s); 2.42 (3H, s); 2.50 (2H, d); 2.85 (1H, dd); 3.00 (1H, dd); 4.12-4.30 (3H); 6.66 (1H, d); 7.02 (1H, d); 7.18-7.40 (3H); 8.29 (1H, d)

Example 9

Preparation of ethyl 1,4-dihydro-6-methyl-2-(N-methyl-N-(2-((2-hydroxy-3-(2-methoxyphenoxy)-propyl)-triphenylmethylamino)-2-methylpropyl)-aminomethyl-4-(3-nitrophenyl)-3,5-pyridinedicarboxylate

A mixture of (-)-N-(2-((2-hydroxy-3-(2-methoxyphenoxy)-propyl)-triphenylmethylamino)-2-methylpropyl)-methylamine (23.4 g; 0.045 moles) prepared as disclosed in example 7, 2-bromoethyl-1,4-dihydro-6-methyl-4-(3-nitrophenyl)-3,5-pyridinedicarboxylate (20.2 g; 0.045 moles) and triethylamine (9.2 ml; 0.066 moles) in toluene (230 ml) is stirred at room temperature for three hours.

The reaction mixture is washed with water (200 ml), with a saturate potassium bicarbonate solution (150 ml) and again with water (100 ml).

After drying over sodium sulfate the organic phase is filtered and the solvent evaporated.

The reaction crude product is purified by chromatography on silics gel column (230-400 mesh), eluent - petroleum ether : ethyl acetate:ethanol: propylamine = 100:100:0.5:1. Ethyl 1,4-dihydro-6-methyl-2-(N-methyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate is obtained.

The chromatographic analysis (thin layer chromatography -eluent, $CH_2Cl_2$:methanol:$NH_4OH$ = 98.2:2:0.2) shows two spots corresponding to the two enantiomers of the two diastereomeric pairs.
Mass spectrum (chemical ionization, negative ions, isobutane): m/e 654 $(M-CPh_3)^-$; 623;415;123.
$^1$H-NMR (300 MHz, $CDCl_3$ ) delta (ppm): 0.90-1.40 (6H, 4s); 1.16-1.26 (6H, m); 2.20 (3H, 2s) 2.28 (3H, s); 2.24-2.40 (2H, m); 2.50-2.70 (2H, m); 3.75 (3H, s); 3.84-4.15 (7H); 5.01 and 5.50 (1H, 2s); 6.30 (1H, bd); 6.70-6.90 (3H, m); 7.20-7.32 (9H, m); 7.47-7.52 (7H); 7.59 (1H, bd); 7.96 (1H, m); 8.11 (1H, bs).

Working in a similar way, the following compounds were prepared:

ethyl 1,4-dihydro-6-methyl-2-(N-isopropyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

(thin layer chromatography - eluent, $CH_2Cl_2$:methanol :$NH_4OH$ =98 :2:0.2, two spots corresponding to the two racemic diastereo isomers)
$^1$H-NMR (300 MHz, $CDCl_3$): delta (ppm): 0.90-1.30 (18H); 2.15-2.40 (5H); 2.60-2.80 (2H, m); 3.50 (1H, dd); 3.70 (2H, bd); 3.74 (3H, s); 5.01 and 5.05 (1H, 2s); 6.50 (1H, bd); 6.80 (3H, m); 7.28 (9H, m), 7.44-7.52 (7H); 7.60 (1H, m); 7.95 (1H, bd); 8.12 (1H, bd).

ethyl 1,4-dihydro-6-methyl-2-(N-butyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

(thin layer chromatography - eluent, $CH_2Cl_2$:methanol:$NH_4OH$ = 98 :2:0.2)
Mass spectrum (chemical ionization, negative ions, isobutane):m/e 936 $(M+1)^-$; 694; 664; 457; 444.
$^1$H-NMR (300 MHz, $CDCl_3$): delta (ppm): 0.82 and 0.84 (3H, 2t); 0.92-1.04 (6H, 4s); 1.16 (2H, m); 1.22 (6H, m); 1.34 (2H, m); 2.18 and 2.21 (3H, 2s; 2.30-2.46 (4H); 2.60 (2H, m); 3.59 (2H, m); 3.75 (3H, s), 3.80-4.12 (7H); 5.01 and 5.05 (1H, 2s); 6.52 (1H, bd); 6.80 (3H, m), 7.25 (9H, m); 7.50 (7H); 7.57 (1H, bd); 7.95 (1H, bd); 8.10 (1H, bs).

ethyl 1,4-dihydro-6-methyl-2-(N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

(thin layer chromatography - eluent, $CH_2Cl_2$:methanol:$NH_4OH$=98: 2:0.2)
Mass spectrum (chemical ionizationn, negative ions, isobutane):m/e 882 $(M)^-$; 640; 402;243;123.
$^1$H-NMR (300 MHz, $CDCl_3$): delta (ppm): 0.93-1.10 (6H); 1.16-1.25 (6H, 2t); 2.15-2.54 (7H); 3.62-3.83 (3H, m); 3.80 (3H, s); 3.98-4.10 (6H, m); 5.08 (1H, bd); 6.64 (1H, bd); 6.76-6.90 (3H, m); 7.20-7.48 (10H); 7.52 (6H, bd); 7.59 (1H, bd); 7.98 (1H, bd); 8.09 (1H, bs); 8.44 (1H, bs).

## Example 10

Preparation of ethyl (-)-1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl-3,5-pyridinedicarboxylate

Concentrate hydrochloric acid (7 ml) is added to a solution of ethyl 1,4-dihydro-6-methyl-2-(N-methyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate (3.4 g; 3.9 mmoles, prepared as described in example 9, in tetrahydrofuran (40 ml). After one hour at room temperature the solvent is evaporated in vacuum at 40 °C.

The crude product is dissolved in methylene chloride (60 ml), washed with an aqueous potassium bicarbonate solution (40 ml) and with water (40 ml).

After drying over soodium sulfate, the organic phase is filtered and the solvent is evaporated.

The crude product is chromatoographed on a silica gel column (230-400 mesh) eluting with petroleum ether:ethyl acetate: ethanol: triethylamine = 10:10:0.2:0.5.

The two levorotatory enantiomers of the two racemic pairs of distereomeric ethyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarbooxylate are thus separated and then converted in their dihydrochloride salts by dissolution in ethyl ether and treatment with an anhydrous saturate solution of HCl in ethyl ether.

Diastereoisomer A (greater Rf) (dihydrochloride), crystalline yellow solid; m.p. = 138-141 °C; $(alpha)_D$ = -15.3° (c = 5%, dimethylsulfoxyde).

Diatereoisomer B (lower Rf) (dihydrochloride), amorphous yellow solid; m.p. = 110-115 °C; $(alpha)_D$ = +5.7 (c = 5%, dimethylsulfoxyde).

Mass spectrum (chemical ionization, positive ions, isobutane): m/e 655 $(M+1)^+$; 625; 531; 238.

Diastereosiomer A:

$^1$H-NMR (free base) (300 MHz, $CDCL_3$): delta (ppm): 1.12 (3H, s); 1.14 (3H, s); 1.20 (3H, t); 1.22 (3H, t); 2.38 and 2.39 (6H, 2s); 2.54 (2H, d); 2.70-2.85 (2H, m); 3.84 (3H, s); 3.91 (2H, s); 3.96-4.14 (7H); 5.06 (1H, s); 6.85-6.98 (4H); 7.35 (1H, t); 7.62 (1H, bd); 7.97 (1H, bd); 8.12 (1H, t).

Diastereoisomer B:

$^1$H-NMR (free base) (300 MHz, $CDCl_3$): delta (ppm): 1.12 (3H, s); 1.14 (3H, s); 1.20 (3H, t); 1.22 (3H, t) 2.38 and 2.39 (6H, 2s); 2.54 (2H, d); 2.70-2.85 (2H, m); 3.84 (3H,s); 3.91 (2H, s); 3.96-4.14 (7H); 5.06 (1H, s); 6.85-6.98 (4H); 7.34 (1H, t); 7.62 (1H, bd); 7.97 (1H, bd); 8.12 (1H, t).

Working in a similar way, the following compounds were prepared:

ethyl 1,4-dihydro-6-methyl-2-(N-isopropyl-N-(2-(2-hydroxy-3-(2-methoxyphenoxy)-propylamino)-2-methylpropyl)-aminomethyl)-4-(3-nitrophenyl)-3,5-pyridinedicarboxylate

Diastereoisomer C (greater Rf) (dihydrochloride), amorphous yellow solid; m.p. = 117-120°C.

Diastereoisomer D (lower Rf) (dihydrochloride), amorphous yellow solid; m.p. = 119-122°C.

Mass spectrum (chemical ionization, positive ions, isobutane):m/e 683 $(M+1)^+$; 653; 560; 444; 432; 238; 201; 125.

$^1$H-NMR (free base) (300 MHz, $CDCl_3$): delta (ppm)

Diastereoisomer C: 1.13 and 1.15 (6H, 2d); 1.11 and 1.12 (6H, 2s); 1.21 and 1.22 (6H, 2t); 2.40 (3H, s); 2.50 (2H, bs); 2.73-2.90 (5H); 3.84 (3H, s); 3.95-4.15 (7H); 5.09 (1H, s) 6.90 (4H, m); 7.33 (1H, t); 7.61 (1H, bd); 7.96 (1H, bd); 8.10 (1H, bs).

Diastereoisomer D: 1.13 and 1.15 (6H, 2d); 1.11 and 1.13 (6H, 2s); 1.21 and 1.22 (6H, 2t); 2.39 (3H, s); 2.51 (2H, dd); 2.73-2.90 (5H); 3.84 (3H, s); 3.95-4.15 (7H); 5.09 (1H, s) 6.90 (4H, m); 7.33 (1H, t); 7.61 (1H, bd); 7.96 (1H, bd); 8.10 (1H, bs).

ethyl 1,4-dihydro-6-methyl-2-(N-butyl-N-(2-(2-hydroxy-3-(2-methoxyphenoxy)-propylamino)-2-methylpropyl)-aminomethyl)-4-(3-nitrophenyl)-3,5-pyridinedicarboxylate

Diastereoisomer E (greater Rf) (dihydrochloride), crystalline yellow solid; m.p. = 108-110 °C.

Ddiastereoisomer F (lower Rf) (dihydrochloride), amorphous yellow solid; m.p. = 113-120 °C

Mass spectrum (chemical ionization, positive ions, isobutane): m/e 697 $(M+1)^+$; 458;446;257;201;125.

$^1$H-NMR (free base) (300 MHz, $CDCl_3$); delta (ppm)

Diasteroisomer E: 0.90 (3H, t); 1.10 and 1.11 (6H, 2s; 1.21 and 1.22 (6H, 2t); 1.30 (2H, m); 1.43 (2H, m); 2.39 (3H, s); 2.50-2.75 (8H); 3.84 (3H, s); 3.90-4.15 (7H); 5.08 (1H, s); 6.90 (4H, m); 7.33 (1H, t); 7.61 (1H, bd); 7.96 (1H, bd); 8.11 (1H, bs):

Diastereoisomer F: 0.90 (3H, t); 1.10 and 1.12 (6H, 2s); 1.21 and 1.22 (6H, 2t); 1.30 (2H, m); 1.43 (2H, m); 2.38 (3H, s); 2.50-2.75 (8H); 3.84 (3H, s); 3.90-4.15 (7H); 5.08 (1H, s); 6.90 (4H, m); 7.33 (1H, t); 7.61 (1H, bd); 7.96 (1H, bd); 8.11 (1H, bs).

Example 11

Preparation of ethyl 1,4-dihydro-6-methyl-2-(N-ethyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

A mixture of ethyl 1,4-dihydro-6-methyl-2-(N-(2-(2-hydroxy-3-(2-methoxyphenoxy)-propyl)-triphenylmethylamino)-2-methylpropyl)-aminomethyl)-4-(3-nitrophenyl)-3,5-pyridinedicarboxylate (20.6 g; 23.4 mmoles), prepared as disclosed in example 9, diethylsulfate (3.3 ml; 25.3 mmoles), triethylamine (5 ml; 34.6 mmoles) and tetrabutylammonium hydrogen sulfate (2.4 g; 7.0 mmoles) in toluene (200 ml) is heated to reflux for 5 hours.

After evaporation of the solvent, the crude product is extracted with methylene chloride (200 ml) and water (100 ml).

The organic phase is separated, dried over sodium sulfate and filtered. By solvent evaporation a crude product is obtained, which is purified by chromatography on silica gel column (230-400 mesh), eluting with petroleum ether:ethyl acetate:propylamine = 120:80:0.3.

Chromatographically pure ethyl 1,4-dihydro-6-methyl-2-(N-ethyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate is thus obtained.

$^1$H-NMR (300 MHz, $CDCl_3$): delta (ppm)0.9-1.5 (9H); 1.18-1.25 (6H, m); 2.18 and 2.22 (3H, 2s); 2.25-2.74 (6H, m); 3.75 (3H, s); 3.78-4.10 (9H, m); 5.01 and 5.06 (1H, 2s); 6.51 (1H, bd); 6.65-6.88 (3H, m); 7.18-7.31 (9H, m); 7.45-7.53 (7H, m); 7.58 (1H, bd); 7.95 (1H, bd); 8.10 (1H, bs); 9.02 and 9.12 (1H, bs).

Mass spectrum (chemical ionization, positive ions, isobutane): m/e 910 $(M)^+$; 669; 417.

Working in a similar way but using allyl iodide and benzyl bromide, respectively, as alkylating agents the following compounds were prepared.

Ethyl 1,4-dihydro-6-methyl-2-(N-2-propenyl-N-(2-(2-hydroxy-3-(2-methoxyphenoxy)-propyl)-triphenylmethylamino)-2-methylpropyl)-aminomethyl)-4-(2-nitrophenyl)-3,5-pyridinedicarboxylate

$^1$H-NMR (300 MHz, $CDCl_3$): delta (ppm): 0.90-1.05 (6H, 4s); 1.16-1.20 (6H, m); 2.18 and 2.24 (3H, 2s); 2.39 (2H, dd); 2.50-2.75 (2H, m); 3.00-3.20 (2H, m); 3.50-4.15 (9H); 3.74 (3H, s); 4.90-5.80 (3H, m); 5.70-5.80 (1H, m); 6.50 (1H, dd); 6.70-6.88 (3H, m); 7.20-7.32 (9H, m); 7.45-7.60 (8H, m); 7.95 (1H, bd); 8.10 (1H, bs).

ethyl 1,4-dihydro-6-methyl-2-(N-benzyl-N-(2-(2-hydroxy-3-(2-methoxyphenoxy)-propyl)-triphenylmethylamino)-2-methylpropyl)-aminomethyl)-4-(3-nitrophenyl)-3,5-pyridinedicarboxylate

$^1$H-NMR (300 MHz, $CDCl_3$): delta (ppm) 0.85-0.95 (6H, m); 2.20 and 2.28 (3H, 2s); 2.30-2.78 (4H); 3.72 and 3.74 (3H, 2s); 3.50-4.10 (11H); 4.98 (1H, bs); 6.46 (1H, m); 6.65-6.86 (3H, m); 7.10-7.30 (16H, m); 7.45-7.54 (6H, m); 7.92 (1H, bd); 8.10 (1H, bd).

Example 12

Preparation of ethyl 1,4-dihydro-6-methyl-2-(N-ethyl-N-(2-(2-hydroxy-3-(2-methoxyphenoxy)-propylamino)-2-methylpropyl)-aminomethyl)-4-(3-nitrophenyl)-3,5-pyridinedicarboxylate

Concentrate hydrochloric acid (7.1 ml) is added to a solution of ethyl 1,4-dihydro-6-methyl-2-(N-ethyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate (3.5 g; 3.9 mmoles), prepared as disclosed in example 11, in tetrahydrofuran (40 ml). After one hour at room temperature the solvent is evaporated in vacuum at 40 °C. The crude solid is dissolved in methylene chloride (60 ml), washed with a potassium bicarbonate saturate aqueous solution (40 ml) and the organic phase is dried over sodium sulfate and filtered.

After evaporation of the solvent, the crude product is purified by chromatography on silica gel column

(230-400 mesh) eluting with petroleum ether:ethyl acetate;triethyl amine : ethanol = 10:10:0.5:0.2.

The two ethyl 1,4-dihydro-6-methyl-2-(N-ethyl-N-(2-(2-hydroxy-3-(2-methoxyphenoxy)-propylamino)-2-methylpropyl)-amino methyl)-4-(3-nitrophenyl)-3,5-pyridinedicarboxylate diastereoisomers are thus separated and then converted in their dihydrochloride salts by treatment with a solution of hydrochloric acid in ethyl ether.

Diastereoisomer G: (greater Rf) (dihydrochloride), amorphous yellow solid; m.p. = 116-118 °C.
Diastereoisomer H: (lower Rf) (dihydrochloride), amorphous yellow solid; m.p. = 117-120 °C.
Mass spectrum (chemical ionization, positive ions, isobutane): m/e 670 $(M+2)^+$; 238.
$^1$H-NMR (free base) (300 MHz, $CDCl_3$): delta (ppm)
Diastereoisomer G: 1.10 and 1.11 (6H, 2s); 1.20 (3H, t); 1.22 (3H, t); 2.40 (3H, s); 2.55 (2H, bs); 2.62 (2H, q); 2.75-2.88 (2H, m); 3.84 (3H, s); 3.90-4.15 (7H); 5.08 (1H, s); 6.86-6.98 (4H, m); 7.33 (1H, t); 7.51 (1H, bd); 7.96 (1H, bd); 8.10 (1H, bs).
Diastereoisomer H: 1.10 and 1.12 (6H, 2s); 1.20 (3H, t); 1.22 (3H, t); 2.40 (3H, s); 2.55 (2H, bs); 2.62 (2H, q); 2.75-2.88 (2H, m); 3.84 (3H, s); 3.90-4.15 (7H); 5.08 (1H, s); 6.86-6.98 (4H, m); 7.33 (1H, t); 7.51 (1H, bd); 7.96 (1H, bd); 8.10 (1H, bs).

Working in a similar way, the following compounds were prepared:

ethyl 1,4-dihydro-6-methyl-2-(N-2-propenyl-N-(2-2-hydroxy-3-(2-methoxyphenoxy)-propylamino)-2-methylpropyl)-aminomethyl)-4-(3-nitrophenyl)-3,5-pyridinedicarboxylate

Diastereoisomer I: (greater Rf)
Diastereoisomer J: (lower Rf)
Both the diastereoisomers I and J (dihydrochloride) melt at 90-95 °C.
Mass spectrum (chemical ionization, positive ions, ammonium hydroxide): m/e 681 $(M+1)^+$; 238.
$^1$H-NMR (free base) (300 MHz, $CDCl_3$): delta (ppm)
Diastereoisomer I: 1.10 and 1.11 (6H, 2s); 1.20 and 1.22 (6H, 2t); 2.40 (3H, s); 2.57 (2H, bs); 2.76 (2H, m); 3.18 (2H, d); 3.85 (3H, s); 3.90-4.15 (9H); 5.08 (1H, s); 5.12 (1H, m); 5.18 (1H, bs); 5.85 (1H, m); 6.84-6.98 (4H); 7.34 (1H, t); 7.11 (1H, bd); 7.97 (1H, dd); 8.11 (1H, bs).
Diastereoisomer J: 1.10 and 1.12 (6H, 2s); 1.20 and 1.22 (6H, 2t); 2.40 (3H, s); 2.57 (2H, bs); 2.76 (2H, m); 3.18 (2H, 2d); 3.85 (3H, s); 3.90-4.15 (9H); 5.08 (1H, s); 5.12 (1H, m); 5.18 (1H, bs); 5.85 (1H, m); 6.84-6.98 (4H); 7.34 (1H, t); 7.11 (1H, bd); 7.97 (1H, dd); 8.11 (1H, bs).

Ethyl 1,4-dihydro-6-methyl-2-(N-benzyl-N-(2-(2-hydroxy-3-(2-methoxyphenoxy)-propylamino)-2-methylpropyl)-aminomethyl)-4-(3-nitrophenyl)-3,5-pyridinedicarboxylate

Diastereoisomer K: (greater Rf)
Diastereoisomer L: (lower Rf)
Both the diastereoisomers K and L (dihydrochloride) melt at 90-95 °C.
Mass spectrum (chemical ionization, positive ions, ammonium hydroxide): m/e 731 $(M+1)^+$; 238
$^1$H-NMR (free base) (300 MHz, $CDCl_3$): delta (ppm):
Diastereoisomer K: 1.04 and 1.05 (6H, 2s); 1.18 and 1.20 (6H, 2t); 2.38 (3H, s); 2.71 (2H, d); 2.79 (2H, d); 3.72 (2H, dd); 3.83 (3H, s); 3.90-4.24 (9H); 5.01 (1H, s); 6.88 (3H, m); 6.95 (1H, m); 7.15-7.34 (6H); 7.52 (1H, bd); 7.95 (1H, bd); 8.10 (1H, bs).
Diatereoisomer L: 1.04 and 1.05 (6H, 2s); 1.18 and 1.20 (6H, 2t); 2.37 (3H, s); 2.71 (2H, d); 2.79 (2H, d); 3.72 (2H, dd); 3.83 (3H, s); 3.90-4.24 (9H); 5.01 (1H, s); 6.88 (3H, m); 6.95 (1H, m); 7.15-7.34 (6H); 7.52 (1H, bd); 7.95 (1H, bd); 8.10 (1H, bs).

## Example 13

### Preparation of methyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarboxylate

A mixture of (-)-N-methyl-2-(2-hydroxy-3-(2-methoxyphenoxy)-propylamino)-2-methylpropylamine (6.4 g, 22.6 mmoles), methyl 2-chloromethyl-1,4-dihydro-6-methyl-4-(3-nitrophenyl)-3,5-pyridinedicarboxylate (9.6

g, 22.6 mmoles) and triethylamine (6.1 ml, 42.3 mmoles) in toluene is maintained at room temperature for 3 hours.

The reaction mixture is washed with water (100 ml), with a saturate solution of potassium bicarbonate (75 ml) and with water (50 ml) again.

The organic phase is dried over sodium sulfate and filtered; the solvent is evaporated. The crude product is purified and the two diastereoisomers are separated by chromatography on silica gel (230-400 mesh) column; eluent, ethyl acetate : toluene : ammonium hydroxide = 90 : 10 : 1. The two diastereoisomer of methyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarboxylate are thus obtained and then treated with a solution of hydrogen chloride gas in ethyl ether to give the corresponding dihydrochloride salts.

Diastereoisomer M: (greater Rf) (dihydrochloride), amorphous yellow solid; m.p. = 138-140 °C; $(alpha)_D$ = -7.5 (c = 0.5%, ethanol).

Diastereoisomer N: (lower Rf) (dihydrochloride), amorphous yellow solid; m.p. = 134-137 °C; $(alpha)_D$ = -4.0 (c = 0.5% ethanol).

Mass spectrum (chemical ionization, positive ions, ammonium hydroxide) m/e 627 $(M+1)^+$; 376; 335; 283; 238.

Diasteroisomer M:

$^1$H-NMR (free base) (300 MHz, $CDCl_3$ ): delta (ppm) 1.12 (6H, s); 2.40 (6H, s); 2.52 (2H, s), 2.76 (2H, m); 3.61 and 3.64 (6H, 2s); 3.83 (3H, s); 3.91 (2H, d); 3.96-4.10 (3H); 5.09 (1H, s); 6.84-6.98 (4H); 7.35 (1H, t); 7.61 (1H, bd); 7.96 (1H, bd), 8.09 (1H, bs).

Diatereoisomer N:

$^1$H-NMR (free base) (300 MHz, $CDCl_3$ ): delta (ppm) 1.11 and 1.13 (6H, 2 s); 2.38 (3H, s); 2.39 (3H, s); 2.53 (2H, dd); 2.78 (2H, m); 3.63 and 3.65 (6H, 2s); 3.84 (3H, s); 3.91 (2H, d); 3.95-4.10 (3H), 5.09 (1H, s); 6.84-6.98 (4H); 7.35 (1H, t); 7.61 (1H, bd); 7.96 (1H, dd); 8.09 (1H, bs).

Working in a similar way, the following compounds were prepared:

isopropyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarboxylate

Diastereoisomer O: (greater Rf) (dihydrochloride), amorphous yellow solid; m.p. = 150-152 °C.

Diastereoisomer P: (lower Rf) (dihydrochloride), amorphous yellow solid; m.p. = 125-128 °C.

Mass spectrum (chemical ionization, positive ions, ammonium hydroxide) m/e 683 $(M+1)^+$; ; 283; 238.

Diastereoisomer O

$^1$H-NMR (free base) (300 MHz, $CDCl_3$ ): delta (ppm) 1.08 (6H, 2d); 1.12 (6H, s); 1.25 (6H, d); 2.37 and 2.39 (6H, 2s); 2.52 (2H, s); 2.80 (2H, m); 3.84 (3H, s); 3.91 (2H, d); 3.98-4.10 (3H), 4.92 (2H, 2m); 5.05 (1H, s) 6.85-7.00 (4H); 7.35 (1H, t), 7.61 (1H, bd); 7.96 (1H, dd); 8.11 (1H, bs).

Diastereoisomer P

$^1$H-NMR (free base) (300 MHz, $CDCl_3$ ): delta (ppm): 1.06-1.14 (12H); 1.24 and 1.26 (6H, 2s), 2.37 and 2.39 (6H, 2s); 2.51 (2H, s); 2.76 (2H, m); 3.84 (3H, s); 3.90 (2H, s); 3.98-4.10 (3H); 4.92 (2H, 2m), 5.04 (1H, s), 6.85-7.00 (4H); 7.33 (1H, t); 7.61 (1H, bd); 7.97 (1H, dd); 8.11 (1H, bs).

2-methoxyethyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-3,5-pyridinedicarboxylate

Diastereoisomer Q: (greater Rf) (dihydrochloride), amorphous yellow solid; m.p. = 125-127 °C.

Diastereoisomer R: (lower Rf) (dihydrochloride), amorphous yellow solid; m.p. = 127-128 °C.

Mass spectrum (chemical ionization, positive ions, ammonium hydroxide) m/e 715 $(M+1)^+$; ; 283; 238.

Diastereoisomer Q

$^1$H-NMR (free base) (300 MHz, $CDCl_3$ ): delta (ppm): 1.12 (6H, s); 2.39 (6H, s) 2.51 (2H, s), 2.77 (2H, m); 3.33 and 3.34 (6H, 2s); 3.54 (4H, m); 3.84 (3H, s); 3.90 (2H, d); 3.95-4.22 (7H); 5.11 (1H, s); 6.84-6.98 (4H); 7.34 (1H, t); 7.66 (1H, bd); 7.96 (1H, dd); 8.11 (1H, bs).

Diastereoisomer R

$^1$H-NMR (free base) (300 MHz, $CDCl_3$ ): delta (ppm): 1.11 and 1.13 (6H, 2s), 2.39 (6H, s), 2.51 (2H, d); 2.75 (2H, m); 3.33 and 3.34 (6H, 2s); 3.54 (4H, m); 3.83 (3H, s); 3.90 (2H, s); 3.97-4.22 (7H); 5.11 (1H, s); 7.34 (1H, t); 7.67 (1H, bd); 7.97 (1H, dd); 8.11 (1H, bs).

methyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-5-carbethoxy-3-pyridinecarboxylate

Diastereoisomer S: (greater Rf) (dihydrochloride), amorphous yellow solid; m.p. = 120-122 °C.
Diastereoisomer T: (lower Rf) (dihydrochloride), amorphous yellow solid; m.p. = 100-102 °C.
Mass spectrum (chemical ionization, positive ions, ammonium hydroxide) m/e 641 $(M+1)^+$; ; 238.
Diastereoisomer S
$^1$H-NMR (free base) (300 MHz, $CDCl_3$ ): delta (ppm) 1.11 (6H, 1); 1.23 (3H, t), 2.36 and 2.37 (6H, 2s); 2.51 (2H, s); 2.75 (2H, m); 3.61 (3H, s); 3.74 (3H, s); 3.90 (2H, d); 3.95-4.15 (5H); 5.08 (1H, s); 6.85-7.00 (4H), 7.34 (1H, t); 7.61 (1H, bd); 7.96 (1H, dd); 8.10 (1H, bs).
Diastereoisomer T
$^1$H-NMR (free base) (300 MHz, $CDCl_3$ ): delta (ppm) 1.11 and 1.13 (6H, 2s); 1.22 (3H, t); 2.36 and 2.38 (6H, 2s); 2.52 (2H dd); 2.75 (2H, m); 3.61 (3H, s); 3.84 (3H, s); 3.90 (2H, s); 3.96-4.15 (5H); 5.08 (1H, s); 6.85-7.00 (4H); 7.34 (1H, t); 7.61 (1H, bd), 7.96 (1H, dd); 8.10 (1H, bs)

ethyl 1,4-dihydro-6-methyl-2-(N-methyl-N-(2-(2-hydroxy-3-(2-methoxyphenoxy)-propylamino)-2-methylpropyl)-aminomethyl)-4-(3-nitrophenyl)-5-carboxymethyl-3-pyridinecarboxylate

Diastereoisomer U: (greater Rf) (dihydrochloride), amorphous yellow solid; m.p. = 139-141 °C.
Diastereoisomer V: (lower Rf) (dihydrochloride), amorphous yellow solid; m.p. = 129-132 °C.
Mass spectrum (chemical ionization, positive ions, ammonium hydroxide) m/e 641 $(M+1)^+$; ; 611; 238.
Diastereoisomer U
$^1$H-NMR (free base) (300 MHz, $CDCl_3$ ): delta (ppm) 1.12 (6H, s); 1.22 (3H, t), 2.38 (6H, s); 2.51 (2H, s); 2.78 (2H, m); 3.64 (3H, s); 3.84 (3H, s); 3.91 (2H, d); 3.95-4.15 (5H); 5.08 (1H, s); 6.84-6.98 (4H); 7.35 (1H, t); 7.61 (1H, d); 7.98 (1H, dd); 8.11 (1H, bs)
Diastereoisomer V
$^1$H-NMR (free base) (300 MHz, $CDCl_3$ ): delta (ppm): 1.12 and 1.13 (6H, 2s); 1.22 (3H, t); 2.37 and 2.38 (6H, 2s); 2.53 (2H, ab); 2.70 (2H, m); 3.64 (3H, s); 3.83 (3H, s); 3.91 (2H, d); 3.96-4.12 (5H); 5.08 (1H, s); 6.84-7.00 (4H); 7.34 (1H, t); 7.61 (1H, bd); 7.98 (1H, dd); 8.11 (1H, bs).

ethyl 1,4-dihydro-6-methyl-2-(N-methyl-N-(2-(2-hydroxy-3-(2-methoxyphenoxy)-propylamino)-2-methylpropyl)-aminomethyl)-4-(2-trifluoromethyl)-3,5-pyridinedicarboxylate

Diastereoisomer W: (greater Rf) (dihydrochloride), amorphous yellow solid; m.p. = 117-120 °C;
Diastereoisomer X: (lower Rf) (dihydrochloride), amorphous yellow solid; m.p. = 113-115 °C;
Mass spectrum (chemical ionization, positive ions, ammonium hydroxide) m/e 678 $(M+1)^+$;; 283; 238.
Diastereodisomer W
$^1$H-NMR (free base) (300 MHz, $CDCl_3$ ): delta (ppm) 1.11 and 1.12 (6H, 2s); 1.16 and 1.17 (6H, 2t); 2.33 and 2.35 (6H, 2s); 2.50 (2H, s); 2.77 (2H, m); 3.84 (3H, s); 3.85 (2H, dd); 3.93-4.20 (7H); 5.60 (1H, bs); 6.86-7.00 (4H); 7.20 (1H, m); 7.37 (1H, bt); 7.46 (1H, bd); 7.51 (1H, bd).
Diastereoisomer X:
$^1$H-NMR (free base) (300 MHz, $CDCl_3$ ): delta (ppm) 1.11 and 1.12 (6H, 2s); 1.16 and 1.17 (6H, 2t); 2.50 (2H, s); 2.77 (2H, m); 3.84 (6H, s) 3.85 (2H, bs) 3.90-4.20 (7H); 5.60 (1H, bs); 6.85-7.00 (4H); 7.20 (1H, m) 7.36 (1H, bt); 7.46 (1H, dd); 7.51 (1H, bd).

ethyl 1,4-dihydro-6-methyl-2-(N-methyl-N-(2-(2-hydroxy-3-(2-methoxyphenoxy)-propylamino)-2-methylpropyl)-aminomethyl)-4-(2-chlorophenyl)-3,5-pyridinedicarboxylate

Diastereoisomer Y: (greater Rf) (dihydrochloride), amorphous yellow solid; m.p. = 115-118 °C;
Diastereoisomer Z: (lower Rf) (dihydrochloride), amorphous yellow solid; m.p. = 108-110 °C;
Mass spectrum (chemical ionization, positive ions, ammonium hydroxide) m/e 644 $(M+1)^+$;; 238.
Diastereoisomer Y:
$^1$H-NMR (free base) (300 MHz, $CDCl_3$ ): delta (ppm) 1.11 (6H, s), 1.19 (6H, bt); 2.32 and 2.35 (6H, 2s); 2.50 (2H, s); 2.75 (2H, m); 3.83 (3H, s); 3.85 (2H, dd); 3.95-4.10 (7H); 5.40 (1H, s); 6.85-7.40 (8H).
Diastereoisomer Z:
$^1$H-NMR (free base) (300 MHz, $CDCl_3$ ): delta (ppm) 1.12 (6H, bs)); 1.19 (6H, t); 2.32 and 2.36 (6H, 2s); 2.51 (2H, s); 2.80 (2H, m); 3.83 (3H, s), 3.86 (2H, bs); 4.00-4.12 (7H); 5.40 (1H, s); 6.85-7.40 (8H).

ethyl 1,4-dihydro-6-methyl-2-(N-methyl-N-(2-(2-hydroxy-3-(2-methoxyphenoxy)-propylamino)-2-methylpropyl)-aminomethyl)-4-(2,3-dichlorophenyl)-3,5-pyridinedicarboxylate

Diastereoisomer AA: (greater Rf) (dihydrochloride), amorphous yellow solid; m.p. = 112-114 °C;
Diastereoisomer AB: (lower Rf) (dihydrochloride), amorphous yellow solid; m.p. = 118-121 °C;
Mass spectrum (chemical ionization, positive ions, ammonium hydroxide) m/e 678 $(M+1)^+$;; 238.
Diastereoisomer AA:
$^1$H-NMR (free base) (300 Mhz, $CDCl_3$ ) delta (ppm) 1.11 (6H, bs); 1.18 (6H, 2t), 2.32 and 2.35 (6H, 2s); 2.50 (2H, s); 2.76 (2H, m); 3.84 (3H, s), 3.85 (2H, dd); 3.96-4.10 (7H); 5.47 (1H, s); 6.85-7.00 (4H); 7.03 (1H, t); 7.22 (1H, dd); 7.29 (1H, dd)
Diastereoisomer AB:
$^1$H-NMR (300 Mhz, $CDCl_3$ ): delta (ppm) 1.11 (6H, 2s); 1.18 (6H, 2t), 2.33 and 2.35 (6H, 2s); 2.50 (2H, s); 2.76 (2H, m), 3.84 (3H, s); 3.85 (2H, bs), 3.98-4.10 (7H); 5.46 (1H, s); 6.85-7.00 (4H), 7.02 (1H, t); 7.22 (1H, bd); 7.29 (1H, dd).

ethyl 1,4-dihydro-6-methyl-2-(N-methyl-N-(2-(2-hydroxy-3-(1-naphthoxy)-propylamino)-2-methylpropyl)-aminomethyl)-4-(3-nitrophenyl)-3,5-pyridinedicarboxylate

Diastereoisomer AC: (greater Rf) (dihydrochloride), amorphous yellow solid; m.p. = 128-131 °C;
Diastereoisomer AD: (lower Rf) (dihydrochloride), amorphous yellow solid; m.p. = 122-125 °C;
Mass spectrum (chemical ionization, positive ions, ammonium hydroxide) m/e 675 $(M+1)^+$;; 643; 258.
Diastereoisomer AC:
$^1$H-NMR (free base) (300 MHz, $CDCl_3$ ): delta (ppm) 1.14 (6H, 2s); 1.19 (6H, 2t); 2.35 and 2.40 (6H, 2s); 2.54 (2H, s); 2.90 (2H, 2m), 3.92 (2H, dd); 4.00-4.22 (7H); 5.07 (1H, s); 6.80 (1H, d); 7.30-8.20 (10 H).
Diastereoisomer AD
$^1$H-NMR (free base) (300 MHz, $CDCl_3$ ): delta (ppm) 1.15 (6H, 2s); 1.20 (6H, 2t); 2.36 and 2.40 (6H, 2s); 2.54 (2H, s); 2.90 (2H, 2m); 3.94 (2H, bd); 4.00-4.22 (7H); 5.08 (1H, s); 6.80 (1H, d), 7.30-8.20 (10H).

ethyl 1,4-dihydro-6-methyl-2-(N-methyl-N-(3-(2-hydroxy-3-(2-methoxyphenoxy)-propylamino)-3-methylbutyl)-aminomethyl)-4-(3-nitrophenyl)-3,5-pyridinedicarboxylate

Diastereoisomers mixture (dihydrochloride), amorphous yellow solid;
Mass spectrum (chemical ionization, positive ions, isobutane) m/e 669 $(M+1)^+$;; 543.
$^1$H-NMR (free base) (300 Mhz, $CDCl_3$ ): delta (ppm) 1.12 and 1.20 (6H, 2s); 1.14-1.25 (6H); 1.60 and 1.72 (2H, 2 m); 2.32 and 2.33 (2H, 2s); 2.38 (3H, s); 2.65 (2H, m); 2.76 (1H, dd); 2.90 (1H, dd); 3.62-3.78 (2H); 3.83 (3H, 2s); 3.98-4.15 (7H); 5.09 and 5.11 (1H, 2s); 6.85-6.98 (4H); 7.35 (1H, bt); 7.60 (1H, bd); 7.98 (1H, dd); 8.10 (1H, m).

ethyl 1,4-dihydro-6-methyl-2-(N-methyl-N-(2-(2-hydroxy-3-(1-carbazolyloxy)-propylamino)-2-methylpropyl)-aminomethyl)-4-(3-nitrophenyl)-3,5-pyridinedicarboxylate

Diastereoisomer AE: (greater Rf) (dihydrochloride), amorphous yellow solid; m.p. = 151-154 °C;
Diastereoisomer AF: (lower Rf) (dihydrochloride), amorphous yellow solid; m.p. = 149-151 °C;
Mass spectrum (chemical ionization, positive ions, ammonium hydroxide) m/e 714 $(M+1)^+$;; 402; 378; 297; 184.
Diastereoisomer AE:
$^1$H-NMR (free base) (300 MHz, $CDCl_3$ ): delta (ppm) 1.16 (6H, bs); 1.20 (6H, 2t); 2.32 (3H, s); 2.39 (3H, s); 2.53 (2H, bs); 2.97 (2H, m); 3.91 (2H, dd); 4.00-4.34 (7H); 5.06 (1H, s); 6.66 (1H, d); 7.06 (1H, d); 7.15-7.44 (5H); 7.61 (1H, bs); 7.95 (1H, dd); 8.11 (1H, bs); 8.22 (1H, d).
Diastereoisomer AF:
$^1$H-NMR (free base) (300 MHz, $CDCl_3$ ): delta (ppm) 1.16 (6H, bs); 1.21 (6H, 2t), 2.34 (3H, s); 2.39 (3H, s); 2.54 (2H, bs); 2.98 (2H, m); 3.91 (2H, bs); 4.00-4.34 (7H); 5.08 (1H, s); 6.66 (1H, d); 7.06 (1H, d); 7.15-7.44 (5H); 7.61 (1H, bs); 7.95 (1H, dd); 8.11 (1H, bs); 8.22 (1H, d).

## Example 14

Preparation of Ethyl 1,4-dihydro-6-methyl-2-(N-acetyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

A mixture of ethyl 1,4-dihydro-6-methyl-2-(N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate (5 g; 5.6 mmoles), prepared according to example 9, acetic anhydride (0.53 ml; 5.6 mmoles), pyridine (0.45 ml; 5.6 mmoles) in toluene (50 ml) is kept at room temperature for two hours.

The reaction mixture is diluted with ethyl acetate (50 ml,) the organic phase is washed with water (30 ml), then with a potassium bicarbonate solution (40 ml) and eventually with water (30 ml).

After anhydrification of the organic extract with sodium sulfate and evaporation of solvent, the crude product is purified by chromatography on silica gel (230-400 mesh) column eluting with ethyl acetate:petroleum ether:ethanol:propylamine = 100:100:0.5:1.

Chromtographically pure Ethyl 1,4-dihydro-6-methyl-2-(N-acetyl-N-(2-((2-hydroxy-3-(2-methoxyphenoxy)-propyl)-triphenylmethylamino)-2-methylpropyl)-aminomethyl)-4-(3-nitrophenyl)-3,5-pyridinecarboxylate is thus obtained (thin layer chromatography; eluent, $CH_2Cl_2$:methanol:$NH_4OH$ = 98:4:0,2).

Mass spectrum (chemical ionization, positive ions, ammonium hydroxide): m/e 681 $(M\text{-}CPh_3)^+$; 342; 243.

IR ($CHCl_3$): characteristic bands at 3300, 3200, 1700, 1650, 1600, 1530, 1500 ($cm^{-1}$).

Working in a similar way but using benzoyl chloride, isobutyryl chloride, ethyl chlorocarbonate and methane sulfonyl chloride as acylating agents, the following compounds were prepared:

Ethyl 1,4-dihydro-6-methyl-2-(N-benzoyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate.

IR ($CHCl_3$): characteristic bands at 3300, 3200, 1690, 1640, 1600, 1530, 1500 ($cm^{-1}$).

Ethyl 1,4-dihydro-6-methyl-2-(N-(1-oxo-2-methylpropyl)-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

Mass spectrum (chemical ionization, positive ions, isobutane): m/e 953 $(M+1)^+$; 710; 458; 342; 243.
IR ($CHCl_3$): bands at 3300, 3220, 1690, 1640, 1600, 1530, 1500 ($cm^{-1}$).

Ethyl 1,4-dihydro-6-methyl-2-(N-ethoxycarbonyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

Mass spectrum (chemical ionization, negative ions, isobutane): m/e 955 $(M+1)^-$; 909; 666; 372; 342.
IR ($CHCl_3$); characteristic bands at 3300, 3200, 1690, 1640, 1600, 1530, 1500 ($cm^{-1}$).

Ethyl 1,4-dihydro-6-methyl-2-(N-methylsulfonyl-N-(2-((2-hydroxy-3-(2-methoxyphenoxy)-propyl)-triphenylmethylamino)-2-methylpropyl)-aminomethyl)-4-(3-nitrophenyl)-3,5-pyridinedicarboxylate

Mass spectrum (chemical ionization, negative ions, isobutane): m/e 881 $(M\text{-}CH_3SO_2)^-$; 718; 638; 401; 372; 243.
IR ($CHCl_3$): characteristic bands at 3320, 3200, 1690, 1650, 1630, 1600, 1530, 1500, ($cm^{-1}$).

Example 15

Preparation of Ethyl 1,4-dihydro-6-methyl-2-(N-acetyl-N-(2-(2-hydroxy-3-(2-methoxyphenoxy)-propylamino)-2-methylpropyl)-aminomethyl)-4-(3-nitrophenyl)-3,5-pyridinedicarboxylate

Concentrate HCl (29 ml) is added to a solution of Ethyl 1,4-dihydro-6-methyl-2-(N-acetyl-N-(2-((2-hydroxy-3-(2-methoxyphenoxy)-propyl)triphenylmethylamino)-2-methylpropyl)-aminomethyl)-4-(3-nitrophenyl)-3,5-pyridinecarboxylate (14.4 g; 15.6 mmoles), prepared as disclosed in example 14, in tetrahydrofuran (150

ml).

After half an hour at room temperature, the solvent is evaporated.

The crude product is extracted with methylene chloride and an aqueous solution of potassium bicarbonate; the organic phase is washed with water and dried over sodium sulfate.

After evaporation of the solvent, the crude product is purified by chromatography on a silica gel column (230-400 mesh) eluting with methylene chloride:methanol:$NH_4OH$ = 100:5:0.5.

Ethyl 1,4-dihydro-6-methyl-2-(N-acetyl-N-(2-(2-hydroxy-3-(2-methoxyphenoxy)-propylamino)-2-methylpropyl)-aminomethyl)-4-(3-nitrophenyl)-3,5-pyridinedicarboxylate is thus obtained in the form of a yellow oil chromatographycally pure (thin layer chromatography; eluent, $CH_2Cl_2$:methanol:$NH_4OH$ = 100:5:0.5).

Hydrochloride: amorphous solid; p.f. = 98-100 °C

Mass spectrum (chemical ionization, positive ions, isobutane): m/e 683 $(M+1)^+$; 665; 648; 575; 508.

$^1$H-NMR (free base) (300 MHz, $CDCl_3$): delta (ppm): 1.15-1.25 (12H) ; 2.05-2.43 (6H, 5s); 2.85 (2H, m); 3.40 (2H, m); 3.82 and 3.84 (3H, 2s); 5.08-5.13 (2H); 6.85-7.00 (4H, m); 7.36 (1H, m), 7.62 (1H, m); 7.98 (1H, m); 8.10 (1H, m).

Working in a similar way, the following compounds were prepared:

Ethyl 1,4-dihydro-6-methyl-2-(N-benzoyl-N-(2-(2-hydroxy-3-(2-methoxyphenoxy)-propylamino)-2-methylpropyl)-aminomethyl)-4-(3-nitrophenyl)-3,5-pyridinedicarboxylate hydrochloride

Mass spectrum (chemical ionization, negative ions, ammonium hydroxide) : m/e 745 $(M+1)^-$; 727; 374; 342.

$^1$HNMR (free base) (300 MHz, $CDCl_3$): delta (ppm): 0.9-1.3 (12H); 1.6 (3H, bs); 3.25 (2H, m); 3.82 and 3.83 (3H, 2s); 5.10-5.50 3H); 6.80-7.00 (4H, m).

Ethyl 1,4-dihydro-6-methyl-2-(N-(1-oxo-2-methylpropyl)-N-(2-(2-hydroxy-3-(2-methoxyphenoxy)-propylamino)-2-methylpropyl)-aminomethyl)-4-(3-nitrophenyl)-3,5-pyridinedicarboxylate hydrochloride

Mass spectrum (chemical ionization, positive ions, isobutane): m/e 711 $(M+1)^+$; 460; 321; 238.

$^1$HNMR (free base) (300 MHz, $CDCl_3$): delta (ppm): 0.96-1.25 (18H); 2.24-2.32 (3H, 3s); 3.10 (1H, m); 3.38 (2H, m); 3.81 and 3.83 (3H, 2s); 5.08-5.16 (3H); 6.90 (4H, m); 7.35 (1H, m); 7.60 (1H, bd); 7.98 (1H, m); 8.10 (1H, bs).

Ethyl 1,4-dihydro-6-methyl-2-(N-ethoxycarbonyl-N-(2-(2-hydroxy-3-(2-methoxyphenoxy)-propylamino)-2-methylpropyl)-aminomethyl)-4-(3-nitrophenyl)-3,5-pyridinedicarboxylate hydrochloride

Mass spectrum (chemical ionization, negative ions, ammonium hydroxide) : m/e 711 $(M-1)^-$; 666; 342.

$^1$HNMR (free base) (300 MHz, $CDCl_3$): delta (ppm): 1.10-1.30 (15H); 2.28-2.40 (3H, m); 2.80 (2H, m); 3.35 (2H, m); 3.82 and 3.84 (3H, 2s); 5.10 (1H, d); 6.90 (4H, m); 7.35 (1H, bt); 7.62 (1H, bd); 7.98 (1H, bd); 8.11 (1H, m).

Ethyl 1,4-dihydro-6-methyl-2-(N-methylsulfonyl-N-(2-(2-hydroxy-3-(2-methoxyphenoxy)-propylamino)-2-methylpropyl)-aminomethyl-4-(3-nitrophenyl)-3,5-pyridinedicarboxylate hydrochloride

Mass spectrum (chemical ionazation, negative ions, isobutane: m/e 719 $(M)^-$; 639; 372; 345.

$^1$H-NMR (free base) (300 MHz, $CDCl_3$): delta (ppm): 1.15-1.25 (12H); 2.38 and 2.40 (3H, 2s); 2.80 (2H, m); 3.00 (3H, bs); 3.19 (2H, m); 3.83 and 3.84 (3H, 2s); 4.56 (1H, dd); 4.88 (1H, d); 5.10 (1H, d); 6.90 (4H); 7.37 (1H, t); 7.66 (1H, bd); 7.98 (1H, bd); 8.12 (1H, bs).

## Example 16

### Evaluation of the pharmacological activity in vitro

Calcium-antagonist activity of the compounds of formula I has been tested as capability of antagonizing the effect of $Ca^{++}$ submaximal concentrations (3mM) on preparations of rabbit mesenteric artery in Krebs

depolarizer, prepared according to Towart "J. Cardiovasc. Pharmacol. 4, 895, (1982)".

The compounds of this invention, tested as calcium antagonist agents on the rabbit mesnteric artery show an $IC_{50}$ of from 10 to 200 (nM); Ki affinity for $beta_1$ receptors is of from 30 to 2000 (nM) and for $beta_2$ receptors is of from 20 to 5000 (nM).

By way of example, $IC_{50}$ of some compounds of formula I are given in the following table 1.

Table 1

| rabbit mesenteric artery test | |
|---|---|
| Compound | $IC_{50}$ (nM) |
| Compound 1 | 47 |
| diastereoisomer A | 15 |
| diastereoisomer B | 120 |

Moreover, the beta-blocking activity has been tested by means of the binding test both towards $beta_1$ heart receptors and towards $beta_2$ lung receptor in mouse according to the methods described in the J. Biol. Chem., 253, 5090, (1978) and in the Br. j. Pharmacol., 68, 57, (1980).

The compounds of formula I showed a greater affinity towards $beta_1$ receptors than towards $beta_2$ receptors.

By way of example, in table 2 the receptorial affinity data as Ki (nM) for some sompounds of formula I are given.

Table 2

| Receptorial affinity | | |
|---|---|---|
| Compound | Ki (nM) mouse heart $beta_1$ receptors | Ki (nM) mouse lung $beta_2$ receptors |
| diastereoisomer A | 600 | 2050 |
| diastereoisomer B | 165 | 560 |
| atenolol | 840 | 18000 |

## pharmacological activity in vivo

The compounds of formula I produce antihypertensive effects in mouse and dog with concomitant reduction of the heart rate.

For example, in the anesthetized dog, compound 1 caused a heart rate reduction of from 9 to 23% and a reduction of the mean arterial pressure of from 3 to 15% at doses of from 100 to 400 ug/kg; at the same doses diastereoisomer A caused a heart rate reduction of from 17 to 30% and a mean arterial pressure reduction of from 12 to 25%, respectively. Both hypotensive effect and bradycardia proved to be particularly durable, lasting over five hours.

## Claims

1. A compound having formula

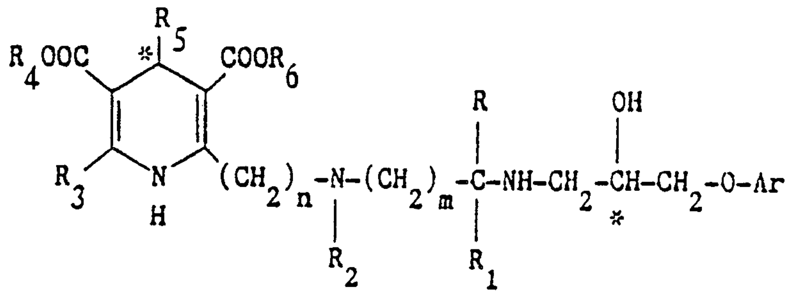

(I)

wherein

Ar is a mono- or dicyclo aromatic or heteroaromatic ring system having from 5 to 10 atoms in the aromatic nucleus optionally substituted by one or more substituents selected from halogen, hydroxy, $C_1$-$C_5$ alkyl, $C_2$-$C_5$ alkenyl, $C_1$-$C_3$ alkoxy, alkoxyalkyl having from 1 to 5 carbon atoms both in the alkyl and in the alkoxy moiety and an optional unsaturation in the chain, phenoxy, phenylalkoxy, aminocarbonylalkyl having from 1 to 3 carbon atoms in the alkyl moiety, cyano, carboxy, carbamoylalkyl, aminocarboxy, amino, mono- and di-alkylamino where the nitrogen atom may be a part of a ring;

R and $R_1$, the same or different, are hydrogen or $C_1$-$C_3$ alkyl;

$R_2$ is a straight or branched $C_1$-$C_7$ alkyl or a straight or branched $C_2$-$C_7$ alkenyl optionally substituted by aryl, additionally $R_2$ may be X-$R_7$ where X is CO, CS or $SO_2$ and $R_7$ is an alkyl or an alkoxyalkyl having from 1 to 5 carbon atoms both in the alkyl and the alkoxy moiety, hydroxy, $C_1$-$C_3$ alkoxy, mono- or di-alkylamino having from 1 to 5 carbon atoms in the alkyl moiety, or $C_1$-$C_5$ alkylthio;

$R_3$ is cyano, amino or $C_1$-$C_3$ alkyl optionally substituted by fluorine atoms;

$R_4$ and $R_6$, the same or different, are alkyl or alkoxyalkyl having from 1 to 5 carbon atoms both in the alkyl and alkoxy moiety;

$R_5$ is a ring selected from phenyl, naphthyl, tetrahydronaphthyl and indanyl wherein said ring may be substituted by one or more substituents selected from halogen, hydroxy, alkyl, alkenyl, alkoxy, alkenyloxy, alkoxyalkyl, alkanoyl, trifluoromethyl, amino, nitro, carbamoyl, cyano, alkylthio, carbamoylalkyl and alkanoylamino having up to 6 carbon atoms in the alkyl moiety;

<u>m</u> and <u>n</u>, the same or different, are 1, 2 or 3;

and the salts thereof with organic or inorganic pharmaceutically acceptable acids.

2. A compound according to claim 1, wherein Ar is a cyclic or bicyclic, aromatic or heteroaromatic ring selected from phenyl, naphthyl, isobenzofuranyl, benzofuranyl, 3,4-dihydrocarbostyryl, benzopyranyl, tetrahydronaphthyl, carbazolyl, indenyl, indolyl and 1,2,5-thiadiazolyl optionally substituted by fluorine, chlorine, bromine, acetyl, allyl, carbamoylmethyl, butyroylamino, cyclohexyl, cyano, hydroxy, butyroyl, acetylamino, methoxycarbonyl, methoxyethyl, methoxy, allyloxy, cyclopentyl, cyclopropyl, morpholine, ethyl and isobutyroyl.

3. A compound according to claim 1 or 2, wherein

Ar is selected from 2-methoxyphenyl, 2-allyloxyphenyl, 2-cyanophenyl, 2-methylphenyl, 2-allylphenyl, 4-carbamoylmethylphenyl, 4-hydroxyphenyl, 4-morpholino-1,2,5-thiadiazol-3-yl, indol-4-yl and 3,4-dihydro-1-(H)-carbostiryl-5-yl;

R and $R_1$, the same or different, are hydrogen or methyl;

$R_2$ has the meanings mentioned above in connection with formula I;

$R_3$ is methyl or trifluoromethyl;

$R_5$ is phenyl substituted by one or more substituents selected from nitro, trifluoromethyl and halogen;

X is a carbonyl or sulfonic group.

$R_7$ is methyl, ethyl, isopropyl, phenyl, dimetylamino, diethylamino, methoxy or ethoxy.

4. A pharmaceutical composition containing a compound according to claim 1 together with a pharmaceutical excipient.

<u>Claims for the following Contracting States: GR, ES</u>

1. A process for preparing a compound of formula

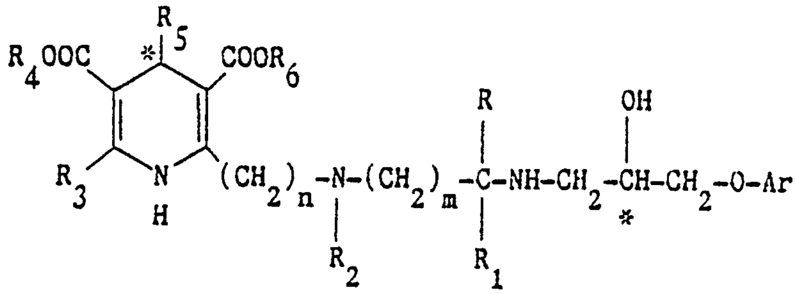

(I)

wherein
Ar is a mono- or dicyclo aromatic or heteroaromatic ring system having from 5 to 10 atoms in the aromatic nucleus optionally substituted by one or more substituents selected from halogen, hydroxy, $C_1$-$C_5$ alkyl, $C_2$-$C_5$ alkenyl, $C_1$-$C_3$ alkoxy, alkoxyalkyl having from 1 to 5 carbon atoms both in the alkyl and in the alkoxy moiety and an optional unsaturation in the chain, phenoxy, phenylalkoxy, aminocarbonylalkyl having from 1 to 3 carbon atoms in the alkyl moiety, cyano, carboxy, carbamoylalkyl, aminocarboxy, amino, mono- and di-alkylamino where the nitrogen atom may be a part of a ring;
R and $R_1$, the same or different, are hydrogen or $C_1$-$C_3$ alkyl;
$R_2$ is a straight or branched $C_1$-$C_7$ alkyl or a straight or branched $C_2$-$C_7$ alkenyl optionally substituted by aryl, additionally $R_2$ may be X-$R_7$ where X is CO, CS or $SO_2$ and $R_7$ is an alkyl or an alkoxyalkyl having from 1 to 5 carbon atoms both in the alkyl and the alkoxy moiety, hydroxy, $C_1$-$C_3$ alkoxy, mono- or di-alkylamino having from 1 to 5 carbon atoms in the alkyl moiety, or $C_1$-$C_5$ alkylthio;
$R_3$ is cyano, amino or $C_1$-$C_3$ alkyl optionally substituted by fluorine atoms;
$R_4$ and $R_6$, the same or different, are alkyl or alkoxyalkyl having from 1 to 5 carbon atoms both in the alkyl and alkoxy moiety;
$R_5$ is a ring selected from phenyl, naphthyl, tetrahydronaphthyl and indanyl wherein said ring may be substituted by one or more substituents selected from halogen, hydroxy, alkyl, alkenyl, alkoxy, alkenyloxy, alkoxyalkyl, alkanoyl, trifluoromethyl, amino, nitro, carbamoyl, cyano, alkylthio, carbamoylalkyl and alkanoylamino having up to 6 carbon atoms in the alkyl moiety;
m and n, the same or different, are 1, 2 or 3;
and the salts thereof with organic or inorganic pharmaceutically acceptable acids
comprising

(i) the condensation of the $R_2$-NH- group of a diamine of formula

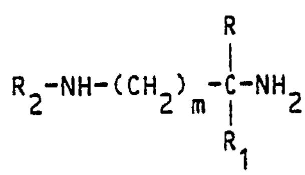

(II)

wherein R, $R_1$, and m have the above mentioned meanings and $R_2$ is a member selected from alkyl and alkenyl wherein both members, straight or branched, have from 1 to 7 carbon atoms and are optionally substituted by an aryl group;
with a 2-alkylen-4-aryl-dihydropyridine and

(ii) the introduction of a 3-aryloxy-2-hydroxy-propyl radical on the primary amino group of (II).

2. A process according to claim 1 characterized in that the primary amino group of diamine II is protected according to conventional techniques before performing step (i) above.

3. A process according to claim 2 characterized in that the protective group is selected from benzyl, benzyloxycarbonyl, ter.butyloxycarbonyl and phthalimido group.

4. A process according to claim 2, characterized in that step (i) comprises condensation of a 2-akylen-4-aryl-dihydropyridine compound with a protected diamine (II) and subsequent removal of the protective group, to give a compound of formula

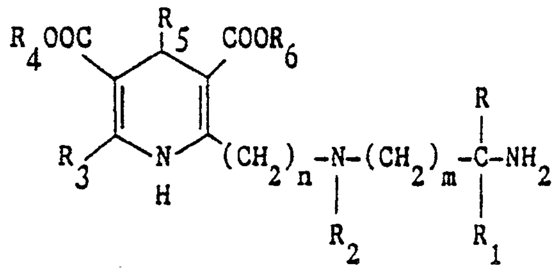 (III)

5. A process according to claim 4, characterized in that step (ii) comprises the condensation of the compound (III) with an epoxide (IV) or with a compound of formula or (V)

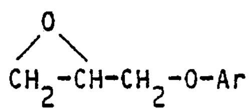 (IV)

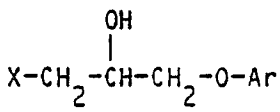 (V)

wherein X is halogen, alkyl or arylsulfonyloxy, to afford a compound of formula I.

6. A process according to claim 1, characterized in that step (i) comprises the condensation of a protected diamine (II) with a 2-alkylen-4-aryl-dihydropyridine compound of formula

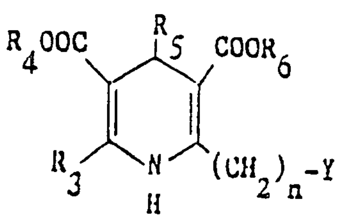 (VI)

wherein Y is a leaving group; in an inert solvent and in the presence of a base.

7. A process according to claim 6, characterized in that the leaving group is selected from chlorine, bromine, iodine, alkylsulfonyloxy, and arylsulfonyloxy.

8. A process according to claim 6, characterized in that the leaving group is selected from chlorine, bromine, iodine, methanesulfonyloxy, benzenesulfonyloxy and p-toluensulfonyloxy.

9. A process according to claim 1, characterized in that step (i) comprises the condensation of a protected diamine (II) with a 2-alkylen-4-aryl-dihydropyridine compound of formula

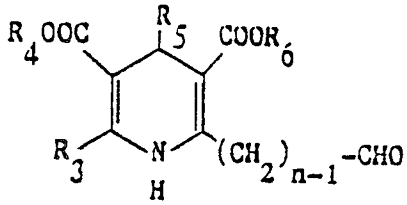 (VII)

in an inert solvent, optionally in the presence of a dehydrating agent, and subsequent reduction.

10. A process according to anyone of the claims from 1 to 9, characterized in that when step (ii) is carried out before step (i), the diamine (II) is optionally protected on the secondary amino group ($R_2$-NH-), the optionally protected diamine (II) is then reacted with a compound of formula (IV) or (V) to give, after

removal of the possible protective group, a compound of formula

$$R_2-NH-(CH_2)_m-\overset{\displaystyle R}{\underset{\displaystyle R_1}{C}}-NH-CH_2-\overset{\displaystyle OH}{CH}-CH_2-O-Ar \qquad (VIII)$$

and finally the compound (VIII) is reacted with a compound of formula (VI) or (VII).